(19) Europäisches Patentamt
European Patent Office
Office européen des brevets

(11) EP 1 839 675 A1

(12) **EUROPEAN PATENT APPLICATION**
published in accordance with Art. 158(3) EPC

(43) Date of publication:
**03.10.2007 Bulletin 2007/40**

(21) Application number: **05785607.2**

(22) Date of filing: **22.09.2005**

(51) Int Cl.:
***A61K 45/00*** *(2006.01)* ***A61K 31/23*** *(2006.01)*
***A61K 31/4545*** *(2006.01)* ***A61K 31/7076*** *(2006.01)*
***A61P 9/00*** *(2006.01)* ***A61P 9/10*** *(2006.01)*
***A61P 43/00*** *(2006.01)* ***C12N 5/06*** *(2006.01)*
***G01N 33/15*** *(2006.01)* ***A61G 13/00*** *(2006.01)*

(86) International application number:
**PCT/JP2005/017494**

(87) International publication number:
**WO 2006/035673 (06.04.2006 Gazette 2006/14)**

(84) Designated Contracting States:
**CH DE FR GB LI**

(30) Priority: **28.09.2004 JP 2004281633**

(71) Applicants:
- **Japan Science and Technology Agency Kawaguchi-shi, Saitama 332-0012 (JP)**
- **National Institutes of Natural Sciences Tokyo 181-8588 (JP)**

(72) Inventors:
- **OKADA, Yasunobu Aichi 444-0871 (JP)**
- **URAMOTO, Hiromi Aichi 444-0846 (JP)**

(74) Representative: **Bittner, Thomas L. et al Forrester & Boehmert Pettenkoferstrasse 20-22 80336 München (DE)**

# (54) NOVEL METHOD OF PROTECTING ISCHEMIC CARDIOPATHY

(57) A method of preventing necrosis in myocardial cells occurring ischemia caused by blood flow injury or post-ischemic reperfusion; and a method of evaluating the necrosis *in vitro* and/or *in vivo.* A pharmaceutical composition for inhibiting cell death in ventricle muscle; a pharmaceutical composition for protecting cardiac muscle in a cardiac surgery and a transplant surgery; and a pharmaceutical composition for preventing a disease caused by necrosis.



Printed by Jouve, 75001 PARIS (FR)

**Description**

TECHNICAL FIELD

The present invention relates to a method of preventing necrosis in myocardial cells occurring ischemia caused by blood flow injury, or post-ischemic reperfusion, and a method of evaluating the necrosis *in vitro* and/or *in vivo.* The invention also relates to a pharmaceutical composition for inhibiting cell death in ventricle muscle, a pharmaceutical composition for protecting cardiac muscle in a cardiac surgery and a transplant surgery, and a pharmaceutical composition for preventing a disease caused by necrosis.

BACKGROUND ART

Necrosis and apoptosis are two types of cell death that occur in myocardial cells following ischemia or post-ischemic reperfusion. Necrosis occurs when ischemia lasts over 45 minutes and when it leaves irreversible damage. The affected area of necrosis spreads over time during ischemia, causing further damage. In order to prevent necrosis from causing further damage, it is necessary to reperfuse the heart as quickly as possible. However, it is known that reperfusion causes abrupt swelling of the myocardial cells and damages cardiac muscle even when it was performed within 20 minutes after ischemia-a time period considered to allow for recovery of myocardial functions (see Non-Patent Publication 1, for example).

It is believed that the damage caused by post-ischemic reperfusion is due to the result of ischemia creating abnormal ion environment in the myocardial cells; however, the mechanism by which this is caused has not been fully understood. In the following, damage caused by abnormalities (for example, cell swelling, edema or disruption of tissues) that occur in myocardial tissues as a result of ischemia will be referred to as "ischemic injury" (for example, cell death, myocardial infarction, and reduced cardiac contractility).

For the prevention of ischemic injury, there has been ongoing development of cardioplegic solution, and research is underway to make modifications to methods of myocardial protection, primarily in the field of cardiac surgery. Specifically, for the protection of cardiac muscle under cardioplegic arrest or during a heart transplant, the following three common methodologies have been established: (1) sudden cardioplegic arrest; (2) surface and local cooling of cardiac muscle; and (3) use of cardioplegic solution as represented by St. Thomas solution (see Non-Patent Publication 2, for example).

Involvement of protein kinase C (PKC) and tyrosine kinase (TK) has been suggested in the myocardial protection as afforded by St. Thomas No. 2 solution. However, contrary to the suspected protective action of TK, there has been report that the size of infarct could be reduced markedly by supplementing St. Thomas No. 2 solution with the TK inhibitor, genistein, and the PKC activator, phorbol 12-myristate 13-acetate (PMA) (Non-Patent Publication 3). However, the reason for this phenomenon has not been elucidated. Further, for the purpose of improving the effect of cardioplegic solution and alleviating ischemic injury, a cardioplegic solution has been supplemented with ATP susceptible $K^+$ channel (KATP channel) opener (see Non-Patent Publications 4 and 5, for example), or $Na^+/H^+$ exchanger (Na/ H exchanger: NHE) blocker (see Non-Patent Publication 6, for example).

Meanwhile, there has been development of ischemic myocardial cardioplegic drugs that combines KATP channel opener and NHE blocker.

It has been known that the cardiac muscle can acquire resistance to ischemia by the procedure known as ischemic preconditioning, which exposes cardiac muscle to brief, repeated periods of ischemia and reperfusion. KATP channel opener is one of the factors that protects against ischemic injury, and it is effectively employed in ischemic preconditioning. However, KATP channel opener exhibits no effect when administered during ischemia or during post-ischemic reperfusion. That is, KATP channel opener is ineffective for injury that has already been caused in affected areas of ischemia. Recently, a large-scale experiment is underway concerning nicorandil, one of the representative KATP channel openers, in an effort to find application of this particular KATP channel opener for the enhancement of heart function after acute myocardial infarction (for example, recovery of a region where cell death has not occurred, or regions surrounding it) (see Non-Patent Publication 7, for example). However, it is not clear as to where and/or how this KATP channel opener acts.

$Na^+/H^+$ exchanger (NHE) is one of the factors involved in ischemic injury. Ischemic injury that involves NHE is induced by activation of NHE as triggered by abnormal cellular ion environment created by ischemia. NHE blocker can exhibit its action when administered during post-ischemic reperfusion or before reperfusion. However, the NHE blocker, cariporide, is ineffective for injury caused by an extended period (45 minutes) of post-ischemic reperfusion (see Non-Patent Publication 8, for example).

There have also been reports that pre-ischemic administration of the phosphodiesterases III (PDE III) inhibitor, milrinone, and its homolog amrinone is also effective in reducing the size of infarct in ischemic cardiac muscle (cardiac muscle exposed to ischemia) and thereby protecting the cardiac muscle (see Non-Patent Publications 9, 10, and 11).

However, these publications do not mention anything about how such protective effects are exhibited on cardiac muscle. Further, application of these substances is severely limited because they do not exhibit any effect when administered after a period of ischemia caused by ischemic heart attack, but are effective only in pre-administration, which needs to be made before an unpredictable heart attack.

As described thus far, there have been known a number of factors that can exhibit protective action against ischemic injury. However, the underlying mechanism of ischemic injury has not been elucidated. As such, no decisive approach has been established for the protection against ischemic injury.

The inventors of the present invention have shown that recovery of cell after reperfusion could be enhanced *in vitro* by the administration of both protein kinase A (PKA) activator and PKC activator during ischemia. Based on observation that administration of cystic fibrosis transmembrane conductance regulator (CFTR) chloride channel blocker is detrimental to cell recovery, the inventors have speculated that the effects of PKA and PKC activators were dues to activation of CFTR channels. (See Non-Patent Publication 12, for example.)

[Non-Patent Publication 1]
J. L. Park & B. R. Lucchesi. Mechanisms of myocardial reperfusion injury. Ann Thorac Surg 68, 1905-1912, 1999
[Non-Patent Publication 2]
H. Furukawa, M. Hachida & K. Yasumoto. Myocardial preservation and reperfusion injury: Current trends and future perspectives. Low Temp Med 26, 93-98, 2000
[Non-Patent Publication 3]
N. Hedayati, S. J. Schomisch, J. L. Carino, J. T. Sherwood, E. J. Lesnefsky & B. L. Cmolik. Cardioprotection by St. Thomas' solution is mediated by protein kinase C and tyrosine kinase. J Surg Bes 113, 121-127, 2003
[Non-Patent Publication 4]
T. Steensrud, D. Nordhaug, O. P. Elvenes, C. Korvald & D. G. Sorlie. Superior myocardial protection with nicorandil cardioplegia. Eur J Cardjothorac Surg 23, 670-677, 2003
[Non-Patent Publication 5]
T. Steensrud, D. Nordhaug, K. V. Husnes, E. Aghajani & D. G. Sorlie. Replacing potassium with nicorandil in cold St. Thomas' Hospital cardioplegia improves preservation of energetics and function in pig hearts. Ann Thorac Surg, 1391-1397, 2004
[Non-Patent Publication 6]
W. J. Linz & A. E. Busch. NHE-1 inhibition: from protection during acute ischaemia/reperfusion to prevention/reversal of myocardial remodelling. Naunyn Schmiedehergs Arch Pharmacol 368, 239-246, 2003
[Non-Patent Publication 7] T. Minamino, K. Jiyoong, M. Asakura, Y. Shintani, H. Asanuma & M. Kitakaze. Rationale and design of a large-scale trial using nicorandil as an adjunct to percutaneous coronary intervention for ST-segment elevation acute myocardial infarction. Japan-working groups of acute myocardial infarction for the reduction of acute myocardial infarction for the reduction of necrotic damage by a K-ATP channel opener (J-WIND-KATP) Circ J 68, 101-106 2004
[Non-Patent Publication 8]
N. N. Aye, S. Komori & K. Hashimoto. Effects and interaction, of cariporide and preconditioning on cardiac arrhythmias and infarction in rat in vivo. Br J Phalmacol 127, 1048-1055, 1999
[Non-Patent Publication 9]
S. Sanada, M. Kitakaze, P. J. Papst, H. Asanuma, K. Node, S. Takashima, M. Asakura, H. Ogita, Y. Liao, Y. Sakata, A. Ogai, T. Fukushima, J. Yamada, Y. Shinozaki, T. Kuzuya, H. Mori, N. Terada & M. Hori. Cardioprotective effect afforded by transient exposure to phosphodiesterase III inhibitors. Circulation 104, 705-710 2001
[Non-Patent Publication 10]
M. P. Rechtman, A. Van der Zypp & H. Majewski. Amrinone reduces ischaemia-reperfusion injury in rat heart. Eur J Pharmacol 402, 255-262, 2000
[Non-Patent Publication 11]
A. F. Rump, D. Acar, W. Klaus. A quantitative comparison of functional and anti-ischaemic effects of the phosphodiesterase-inhibitors, amrinone, milrinone and levosimendan in rabbit isolated hearts. Br J Pharmacol 112, 757-762, 1994
[Non-Patent Publication 12]
H. Uramoto, S. Morishima, Y. Ando-Akatsuka, Y. Nagasaki, N. Hagiwara and Y. Okada. A role of CFTR Cl- channel in the protection from ischemic injury in neonatal rat ventricular myocytes. Jpn. J. Physiol. 52, S35, 2002

Ischemic preconditioning is an effective means to alleviate ischemic injury. However, ischemic preconditioning is not a realistic option because it requires pre-exposure of cardiac muscle to brief, repeated periods of ischemia and reperfusion. Experimentally, drug preconditioning (nicorandil) has been performed. However, this is only effective in preoperative and intraoperative administration, and no effect can be expected when administration was made after the operation or reperfusion. Further, patients under drug preconditioning are usually taking drugs on a daily basis as a preventative measure. That is, preconditioning is intended for preoperative administration, and it serves no purpose as a treatment of patients who have had ischemia. Accordingly, there is a strong demand for factors that are effective for preventing ischemic injury in patients suffering from unexpected ischemia.

Heart disease is one of the most common cause of death in Japan, and it is therefore important and urgent to develop methods for prevention and/or remedy of cell death in myocardial cells caused by ischemia or post-ischemic reperfusion, which may lead to myocardial infarction or other failures. One specific clinical example of myocardial protection is the protection of cardiac muscle in the field of cardiac surgery; namely, protection of cardiac muscle with a cardioplegic solution during open heart surgery. Among several administration methods of cardioplegic solution, a "cardioplegia using a cardioplegic solution with warm blood" (warm blood cardioplegia) has caught attentions for its ability to overcome drawbacks of the hypothermic method and enable early physiological and functional recovery of the cardiac muscle. However, owning to the fact that the procedure is performed under ordinary temperature, it can aggravate cardiac muscle injury by causing edema that results from uneven exposure of cardiac muscle to perfusion. For this reason, this method is only applicable to short surgery. Under these circumstances, development of method that prevents ischemic injury during an open heart surgery will be highly beneficial for the advancement of myocardial protection method.
The present invention was made in view of the foregoing problems, and an object of the invention is to realize a method for prevention and/or remedy of cell death in myocardial cells that occurs during ischemia or post-ischemic reperfusion, and particularly a method for improving conventional myocardial protection methods intended for ischemic injury that occur during open heart surgery, and development of an ischemic injury inhibitor. An object of the present invention is to provide a method for preventing cell death in myocardial cells caused by ischemic injury, intended for internal treatment after the onset (episode) of ischemia in disorders such as angina, and a therapeutic drug intended for use as a cardioplegic solution. An object of the present invention is to provide a method for protecting cardiac muscle from injury caused by ischemia and post-ischemic reperfusion, which are inevitable in cardiac surgery and heart transplant.

DISCLOSURE OF INVENTION

A system for screening a CFTR activator *in vivo* according to the present invention includes: a heat-insulating device for maintaining body temperature; a ligature holder; and a continuous drug-infusion instrument.
In a system according to the present invention, the heat-insulating device is preferably a heated surgery table using a constant temperature bath.
In a system according to the present invention, the ligature holder may be an outer portion of an indwelling needle, and a part of the ligature holder in contact with a ligated portion is preferably made of a material with some flexibility, and is fixed to the ligated portion with a small piece of cloth, 0.5 mm thick, in between.
In a system according to the present invention, the continuous drug-infusion instrument is preferably a transjugular infusion instrument.
It is preferable that a system according to the present invention further includes an instrument for perfusing a heated physiological saline in the thoracic cavity so as to maintain an external environment and temperature of the heart and prevent drying of the heart.
A method according to the present invention is for screening a CFTR activator *in vivo* using a system of the present invention and includes: the steps of: administering a candidate factor of CFTR activator to a subject, either at a start of reperfusion or immediately before or after reperfusion (raising a level of candidate factor to effective level at an affected area at the start of reperfusion); and measuring a size of infarct occurring in the subject.
In a screening method according to the present invention, the administration step is preferably performed transjugularly.
It is preferable that a screening method according to the present invention include the step of comparing the size of infarct occurring in the subject with a size of infarct that has occurred by co-application of a PKA activator and a PKC activator, used instead of the candidate factor in the administration step.
In a screening method according to the present invention, the PKA activator is preferably milrinone.
It is preferable that a screening method according to the present invention include the step of comparing a size of infarct measured in subject wild type mice with a size of infarct measured in subject CFTR knockout mice.
An *in vivo* system for screening a CFTR activator according to the present invention may be an *in vivo* system for ischemia and reperfusion, and includes means for administering a candidate factor of CFTR activator to a subject, either at the start of reperfusion, or immediately before or immediately after reperfusion.
In an *in vivo* system for screening a CFTR activator according to the present invention, the administration means is preferably a continuous drug-infusion instrument.
It is preferable that an *in vivo* system for screening a CFTR activator according to the present invention further includes means for measuring a size of infarct occurring in a subject.
A pharmaceutical composition according to the present invention is for suppressing cell death in ventricle muscle, and includes a CFTR channel activator to this end.
In a pharmaceutical composition according to the present invention, the cell death is preferably necrosis.
In a pharmaceutical composition according to the present invention, the cell death is preferably caused by ischemia or post-ischemic reperfusion.

In a pharmaceutical composition according to the present invention, the CFTR channel activator is preferably a mixture of PKA activator and PKC activator.

In a pharmaceutical composition according to the present invention, the PKA activator is preferably dibutyryl cyclic AMP.

In a pharmaceutical composition according to the present invention, the PKC activator is preferably phorbol 12-myristate 13-acetate.

In a pharmaceutical composition according to the present invention, the CFTR channel activator is preferably a mixture of phosphodiesterase III inhibitor and PKC activator.

In a pharmaceutical composition according to the present invention, the phosphodiesterase III inhibitor is preferably 1,6-dihydro-2-methyl-6-oxo[3,4'-bipyridine]-5-carbonitrile.

In a pharmaceutical composition according to the present invention, the cell death is preferably induced by abnormalities that are caused during ischemia in myocardial tissues.

In a pharmaceutical composition according to the present invention, the cell death preferably accompanies a reduction in activity of mitochondrion dehydrogenase.

A pharmaceutical composition according to the present invention is preferably used to suppress cell death in a primary culture of ventricular myocytes.

In a pharmaceutical composition according to the present invention, the cell death is preferably necrosis.

A pharmaceutical composition according to the present invention preferably reduces an infarct area in ventricle muscle.

A pharmaceutical composition according to the present invention is for protecting cardiac muscle in cardiac surgery and heart transplant, and includes a CFTR channel activator to this end.

A pharmaceutical composition according to the present invention is preferably used in cardiac surgery or heart transplant of a necrotic heart.

In a pharmaceutical composition according to the present invention, the CFTR channel activator is preferably a mixture of PKA activator and PKC activator.

In a pharmaceutical composition according to the present invention, the PKA activator is preferably dibutyryl cyclic AMP.

In a pharmaceutical composition according to the present invention, the PKC activator is preferably phorbol 12-myristate 13-acetate.

In a pharmaceutical composition according to the present invention, the CFTR channel activator is preferably a mixture of phosphodiesterase III inhibitor and PKC activator.

In a pharmaceutical composition according to the present invention, the phosphodiesterase III inhibitor is preferably 1, 6-dihydro-2-methyl-6-oxo [3, 4'-bipyridine] -5-carbonitrile.

A pharmaceutical composition according to the present invention is for preventing diseases induced by necrosis, and includes a CFTR channel activator to this end.

In a pharmaceutical composition according to the present invention, the CFTR channel activator is preferably a mixture of PKA activator and PKC activator.

In a pharmaceutical composition according to the present invention, the PKA activator is preferably dibutyryl cyclic AMP.

In a pharmaceutical composition according to the present invention, the PKC activator is preferably phorbol 12-myristate 13-acetate.

In a pharmaceutical composition according to the present invention, the CFTR channel activator is preferably a mixture of phosphodiesterase III inhibitor and PKC activator.

In a pharmaceutical composition according to the present invention, the phosphodiesterase III inhibitor is preferably 1,6-dihydro-2-methyl-6-oxo[3,4'-bipyridine]-5-carbonitrile.

A method for suppressing cell death in ventricle muscle according to the present invention includes the step of administering a CFTR channel activator.

A method for protecting cardiac muscle in cardiac surgery according to the present invention includes the step of administering a CFTR channel activator.

A method for preventing diseases induced by necrosis according to the present invention includes the step of administering a CFTR channel activator.

A system for inducing ischemia and post-ischemic reperfusion *in vivo* according to the present invention includes a heat-insulating device for maintaining body temperature.

A system for inducing ischemia and post-ischemic reperfusion according to the present invention preferably includes a ligature holder and a continuous drug-infusion instrument.

In a system for inducing ischemia and post-ischemic reperfusion according to the present invention, the heat-insulating device preferably includes a heated surgery table using a constant temperature bath.

A system for inducing ischemia and post-ischemic reperfusion according to the present invention further includes an instrument for perfusing a heated physiological saline in the thoracic cavity so as to maintain an extracellular environment and temperature of the heart and prevent drying of the heart.

In a system for inducing ischemia and post-ischemic reperfusion according to the present invention, the ligature holder is preferably an outer portion of an indwelling needle.

A method for evaluating cell death according to the present invention is for evaluating cell death *in vitro* in a cultured cell system, which occurs as a result of ischemia and post-ischemic reperfusion, and to this end includes:

a step of preparing target cells of evaluation;
an ischemia-treatment step of culturing the cells under anaerobic conditions in glucose-free medium;
a reperfusion-treatment step of culturing the treated cells under aerobic conditions in glucose-containing medium; and
a step of detecting cell death.

In a method for evaluating cell death according to the present invention, it is preferable that the target cells of evaluation be myocardial cells, brain nerve cells, or brain glial cells.

In a method for evaluating cell death according to the present invention, it is preferable that the step of evaluating cell death use changes in morphology of the nucleus as a measure.

A screening method according to the present invention is for *in vitro* screening of a suppressing factor of cell death caused by ischemia and post-ischemic reperfusion, and to this end includes:

a step of preparing cells;
an ischemia-treatment step of culturing the cells under anaerobic conditions in glucose-free medium;
a reperfusion-treatment step of culturing the treated cells under aerobic conditions in glucose-containing medium;
a step of detecting and evaluating cell death; and
a step of comparing a result of evaluation which was obtained by supplementing the medium with a candidate factor in the ischemia-treatment step, the reperfusion-treatment step, or before or after these steps, with a result of evaluation which was obtained without the candidate factor.

In a screening method according to the present invention, it is preferable that the cells be myocardial cells, brain nerve cells, or brain glial cells.

In a screening method according to the present invention, it is preferable that the step of measuring cell death use activity of mitochondrion dehydrogenase, fragmentation of intranuclear DNA, caspase activity, release of cytochrome c from mitochondria to the cytoplasm, or changes in morphology of the nucleus as a measure.

Additional objects, features, and strengths of the present invention will be made clear by the description below. Further, the advantages of the present invention will be evident from the following explanation in reference to the drawings.

BRIEF DESCRIPTION OF DRAWINGS

Figure 1(A) is a graph representing viability of cultured ventricular myocytes of rats, when $Cl^-$ channel blocker 5-nitro-2-2(3-phenylpropylamino)-benzoate (NPPB) was applied to medium during ischemic treatment; and Figure 1 (B) represents a graph representing viability of cultured ventricular myocytes of rats, when NPPB was applied to medium during ischemic treatment, 1 hour prior to ischemic treatment, and for 18 hours after the start of reperfusion.

Figure 2 is a graph representing viability of cultured ventricular myocytes of rats, when CFTR channel activator (N6,2'-O-dibutyriladenosine-3':5'-cyclic monophosphate (dbcAMP) or PMA, or both (DP)) and/or CFTR channel blocker (NPPB, glibenclamide, gemfibrozil, or anthracene-9-carboxylate (9AC)) were administered to medium during ischemia treatment.

Figure 3 is a graph representing viability of cultured ventricular myocytes of rats, when CFTR channel activator (milrinone, isoproterenol, and PMA used together (MIP)) and/or CFTR channel blocker (NPPB, glibenclamide, gemfibrozil, or 9AC) were administered to medium during ischemia treatment.

Figure 4(A) is a photographic view of cultured ventricular myocytes of rats, when the cells, supplemented with CFTR channel blocker (NPPB) in medium during ischemic treatment, were stained with Hoechst 33342 and/or propidium iodide (PI); and Figure 4(B) is a graph representing percentages of PI stained cells.

Figure 5(A) is a graph representing scores for nuclear DNA fragmentation immediately after ischemia, when CFTR channel activator (dbcAMP and PMA used together) or CFTR channel blocker (NPPB) was applied to culture medium of ventricular myocytes of rats during ischemic treatment; and Figure 5(B) is a graph representing scores for nuclear DNA fragmentation 96 hours after post-ischemic reperfusion, when CFTR channel activator (dbcAMP and PMA used together) or CFTR channel blocker (NPPB) was applied to culture medium of ventricular myocytes of rats during ischemic treatment.

Figure 6(A) is a photographic view showing an overall structure of a heat insulating system operating on a mouse; and Figure 6(B) is a photographic view showing how heated physiological saline is supplied to and collected from the thoracic cavity.

Figure 7(A) is a photographic view showing how the left descending coronary artery of a mouse is ligated using a ligature holder during operation; Figure 7(B) is a photographic view showing a heart before ischemia by ligature; and Figure 7(C) is a photographic view showing a heart after ischemia by ligature.

Figure 8(A) is a photographic view showing how a MicroFil™ is inserted into the right jugular venous and fixed thereon for continuous administration of a drug to a mouse; and Figure 8(B) is an enlarged photographic view of Figure 8(A).

Figure 9(A) is a diagram showing administration times of CFTR channel activator (milrinone or PMA, or both) to C57BL/6J mice; Figure 9(B) is a graph representing a proportion of infarct region with respect to ischemic region, when CFTR channel activator was administered in one shot (milrinone 50 μg/kg + PMA 4.8 μg/kg) 10 minutes prior to the start of ischemic treatment, and when this was followed by continuous administration (milrinone 17 μg/kg/h + PMA 1.6 μg/kg/h), which extended from the start to end of ischemic treatment; and Figure 9(C) is a photographic view showing regions of viable cells in heart sections of mice (2,3,5-triphenyltetrazoliumchloreide (TTC) staining).

Figure 10(A) is a diagram showing administration times of CFTR channel activator (milrinone+PMA) to C57BL/6J mice (A and B being continuous administration following one shot administration, and C and D being one shot administration); and Figure 10(B) is a graph representing a proportion of infarct region with respect to ischemic region (one shot administration being made in milrinone 50 μg/kg + PMA 4.8 μg/kg, and continuous administration being made in milrinone 17 μg/kg/h + PMA 1.6 μg/kg/h).

Figure 11(A) is a diagram showing periods of ischemia and reperfusion in experiment using CFTR knockout mice (+/- or +/+); and Figure 11(B) is a graph representing a proportion of infarct region with respect to ischemic region in experiment using CFTR knockout mice (+/- or +/+).

## BEST MODE FOR CARRYING OUT THE INVENTION

The inventors of the present invention diligently worked to solve the foregoing problems. Specifically, the inventors made the following assessments based on a previous result obtained from a primary culture system of neonatal rat ventricular myocytes (*in vitro* experiment system), i.e., based on the fact that post-reperfusion cell recovery can be ameliorated by application of PKA activator and PKC activator during ischemia. More specifically, in addition to the *in vitro* experiment system, the inventors used an *in vivo* experiment system using C57BL/6J mice to assess whether:

(i) the protection mechanism against ischemic injury in myocardial cells afforded by co-activation of PKA and PKC *in vitro* is attributed to actions of CFTR;
(ii) actions of CFTR in the protection mechanism against myocardial cell ischemic injury are associated with protection against which type of cell death, necrosis or apoptosis;
(iii) administration of CFTR channel activator to mice in *vivo* inhibits cardiac muscle injury caused by ischemia or reperfusion, particularly necrotic cell death in myocardial cells;
(iv) administration of CFTR channel activator *in vivo* after the start of ischemia inhibits cardiac muscle injury caused by ischemia or reperfusion, particularly necrotic cell death in myocardial cells; and
(v) the expression level of CFTR *in vivo* influences the degree of ischemic injury in cardiac muscle.

Cystic fibrosis transmembrane conductance regulator (CFTR), which is known to play a role in the secretion of $Cl^-$-containing fluids (reabsorption in the case of sweat glands) in many types of epithelial tissues, is one kind of C1 channel, and the gene encoding CFTR is known as a causal gene of cystic fibrosis. CFTR is one class of ABC transporters (ATP-binding cassette transporters: ATP-binding cassette transporter proteins) with two ATP binding sites. CFTR serves as a $Cl^-$ channel whose control is cAMP-dependent. It also functions as a regulator for other channels.

CFTR is expressed in the exocrine glands throughout the body, such as the digestive glands, trachea, and sweat glands. Functional abnormalities in CFTR are detected as a rise in Cl ion level in the sweat. Recently, the CFTR gene has been identified as a causal gene of not only cystic fibrosis but other types of genetic diseases as well, such as chronic pancreatitis, chronic lower airway disease, sinusitus, and congenital male sterility, suggesting involvement of CFTR gene in a wide range of disorders (Naruse, S., Kitagawa, M., Ishiguro, H., Fujiki, K. & Hayakawa, T.: Cystic fibrosis and related diseases of the pancreas. Best Pract. Res. Clin. Gastroenterol., 16 : 511-526, 2002).

Other than epithelial tissues, CFTR is also expressed in cardiac muscle. This suggests the possibility that CFTR might be involved in the mechanism of cell death in myocardial cells (necrosis, in particular) caused by ischemia or reperfusion. However, a method for preventing ischemic cell death in myocardial cells, targeting at CFTR, has not been developed.

As a result of the foregoing assessments, the inventors of the present invention have found, from a pharmaceutical standpoint, that the enhancement of cell viability by the co-application of PKA activator and PKC activator (dibutyryl cyclic AMP (dbcAMP) and phorbol 12-myristate 13-acetate (PMA) used together) in the conventional *in vitro* ischemia/reperfusion experiment system was due to the activation of the CFTR channel, and that this effect could not be obtained from pre-ischemic or post-ischemic administration alone but, in the *in vitro* experiment system, depended heavily on administration of CFTR channel activator during ischemia.

PMA is known to exhibit a preconditioning-like effect by itself. Substances like PMA that exhibit the preconditioning effect are able to render cardiac muscle resistant to ischemia, by exposing the cardiac muscle to brief, repeated periods of ischemia and reperfusion, prior to ischemia. However, they have no effects on the cardiac muscle of patients already suffering from ischemia. A number of such substances are known that can render cardiac muscle resistant to ischemia by the preconditioning effect. However, no substance is known that can exhibit its effect in post-ischemic administration.

The inventors of the present invention have found that ischemic injury (for example, cell death) could be suppressed by applying CFTR channel activator to myocardial cells *in vitro* during ischemia and activating the CFTR channel. Changes in cell viability were consistent with the death rate; however, there was no involvement of apoptosis and the changes were all due to necrosis. Administration of CFTR channel activator in the ischemia/reperfusion system *in vivo* also showed a marked reduction in infarct size, when the CFTR channel activator was applied prior to ischemia and continuously till the end of the ischemic period, and when it was applied at the start of reperfusion. These observations suggest that the CFTR channel activator, which is applied either prior to ischemia and continuously till the end of the ischemic period, or at the start of reperfusion, is supplied to the affected region with blood immediately following reperfusion, and thereby suppresses ischemic injury.

Based on this finding, the inventors accomplished the present invention by developing a method for preventing ischemic necrosis in myocardial cells, through administration of CFTR channel activator.

A used herein, "CFTR" is used interchangeably with "CFTR channel" or "CFTR chloride channel."

As used herein, "myocardial cells" may be myocardial cells in the body, commercially available cultured cells originating in cardiac muscle, or a primary culture of myocardial cells originating in heart removed from the body.

As used herein, the "ischemia/reperfusion experiment system" is intended to mean an experiment system that can initiate ischemia (or removal of oxygen and glucose) and post-ischemic reperfusion (reoxygenation and re-supply of glucose) in cultured cells or mice, in an *in vitro* system using cultured cells or an *in vivo* system using mice.

(1) Pharmaceutical Composition

The present invention provides a pharmaceutical composition for suppressing necrotic cell death. Necrotic cell death occurs in various parts of body and causes a wide range of disorders. It would be highly advantageous to develop a means for suppressing such cell death, because it makes it possible to provide therapeutic or preventative drugs that are effective for many types of disorders caused by cell death.

A pharmaceutical composition according to the present invention includes a CFTR channel activator. In one embodiment, the CFTR channel activator preferably comprises a protein kinase A (PKA) activator and/or a protein kinase C (PKC) activator, and more preferably comprises a combination of PKA activator and PKC activator. The PKA activator is preferably dbcAMP. The PKC activator is preferably PMA. Instead of PKA activator, phosphodiesterase III inhibitor may be used. A preferable example of phosphodiesterase III inhibitor is milrinone.

In a preferred embodiment, a pharmaceutical composition according to the present invention can be used to suppress cell death in ventricle muscle caused by ischemia or post-ischemic reperfusion. Such cell death in ventricle muscle caused by ischemia or post-ischemic reperfusion can occur in myocardial cells during a heart transplant or as a result of myocardial infarction. The cause of such cell death is known to be either necrosis (as used herein, the term "necrosis" is used interchangeably with necrotic cell death) or apoptosis (as used herein, the term "apoptosis" is used interchangeably with apoptotic cell death). A pharmaceutical composition according to the present embodiment is preferably used to suppress necrosis in ventricle muscle that occurs due to ischemia or post-ischemic reperfusion. More preferably, a pharmaceutical composition according to the present embodiment can be used to suppress necrosis following abnormalities caused by ischemia in myocardial tissues. Non-limiting examples of such ischemia-induced abnormalities include cell swelling, edema and disruption of tissues.

As used herein, the term "ischemia" is intended to mean local hypoemia. Ischemia brings about various kinds of alterations (for example, necrosis) in affected tissues, depending upon such factors as duration of ischemia, susceptibility of tissues to ischemia, and the presence or absence of vascular anastomosis in the affected region. As used herein, "ischemic injury" is intended to mean injury caused by abnormalities that have occurred in myocardial tissues as a result of ischemia. As used herein, "abnormalities occurring during ischemia" or "various abnormalities caused by ischemia in myocardial tissues" mean cell swelling, edema and disruption of tissues, for example. Generally, ischemia causes a wide range of abnormalities in myocardial tissues. Specific examples of ischemic injury include cell death in myocardial cells and attenuation of heart contractility. Injury caused by post-ischemic reperfusion is another example of ischemic injury. The disease (disorder) to be treated by a pharmaceutical composition according to the present invention may be only one kind of ischemic injury or a combination of different kinds of ischemic injury as exemplified above, or even one or more kinds of ischemic injury involving other diseases or disorders.

Cell death can be evaluated using activity of mitochondrion dehydrogenase as a measure. Activity of mitochondrion dehydrogenase is known to weaken following cell death. Thus, a reduction in the enzyme activity can be regarded as a high cell death rate.

A person ordinary skill in the art will readily understand that the CFTR channel activator is effective not only for cell death caused by ischemia or post-ischemic reperfusion but for necrotic cell death having other causations.

The present invention also provides a pharmaceutical composition for protecting cardiac muscle in cardiac surgery. As used herein, "protecting cardiac muscle" or "protection of cardiac muscle" means protection of cardiac muscle in the field of cardiac surgery, or more specifically protection of cardiac muscle with a cardioplegic solution during an open heart surgery or a heart transplant. A pharmaceutical composition according to the present invention is preferably used in cardiac surgery and heart transplant in which necrosis may occur.

Further, the present invention provides a pharmaceutical composition for preventing diseases caused by necrosis. Myocardial infarction is a non-limiting example of "diseases caused by necrosis."

A pharmaceutical composition according to the present invention is applicable to mammals such as humans, mice, rats, rabbits, dogs, cats, cattle, horses, pigs, and monkeys.

A pharmaceutical composition according to the present invention may solely comprise CFTR channel activator (preferably a combination of PKA activator and PKC activator), but may additionally include a pharmaceutically acceptable carrier. A pharmaceutical composition according to the present invention can be produced according to known means in producing methods of pharmaceutical compositions.

In one embodiment, a pharmaceutical composition according to the present invention may be a medical composition. A pharmaceutically acceptable carrier used in such medical compositions can be selected according to administration form or dosage form of the medical composition.

As used herein, pharmacologically acceptable carriers are various kinds of organic and inorganic carrier substances that can be used as material of the drug. Such carriers are contained as a diluting agent, a lubricant, a binder, or a disintegrant in the case of solid drugs. In liquid drugs, the carriers are contained as a solvent, a solubilizing agent, a suspension, an isotonic agent, a buffer, or a soothing agent.

Examples of a diluting agent include: lactose, saccharose, D-mannitol, xylitol, sorbitol, erythrytol, starch, and crystallized cellulose. Examples of a lubricant include: magnesium stearate, calcium stearate, talc, and colloid silica.

Examples of a binder include: pregelatinized starch, methyl cellulose, crystallized cellulose, saccharose, D-mannitol, trehalose, dextrin, hydroxypropylcellulose, hydroxypropyl methylcellulose, and polyvinyl pyrrolidone.

Examples of a disintegrant include: starch, carboxymethyl cellulose, low-substitution hydroxypropylcellulose, carboxymethylcellulose calcium, croscarmellose sodium, and sodium carboxymethyl starch. Examples of a solvent include: injection solvent, alcohol, propylene glycol, macrogol, sesame oil, corn oil, and tricaprylin.

Examples of a solubilizing agent include: polyethylene glycol, propylene glycol, D-mannitol, trehalose, benzyl benzoate, ethanol, tris aminomethane, cholesterol, triethanolamine, sodium carbonate, and sodium citrate.

Examples of a suspension include: detergents such as stearyl triethanolamine, sodium lauryl sulfate, lauryl amino propionic acid, lecithin, benzalkonium chloride, benzethonium chloride, and glyceryl monostearate; and hydrophilic high-molecular-weight molecules such as polyvinyl alcohol, polyvinyl pyrrolidone; carboxymethylcellulose sodium, methyl cellulose, hydroxymethyl cellulose, hydroxyethyl cellulose, and hydroxypropylcellulose.

Examples of an isotonic agent include: sodium chloride, glycerine, and D-mannitol.

Examples of a buffer include: phosphate, acetate, carbonate, and citrate.

Examples of a soothing agent include benzyl alcohol.

Examples of an antiseptic include: parahydroxybenzoate esters, chlorobutanol, benzyl alcohol, phenethyl alcohol, dehydroacetic acid, and sorbic acid.

Examples of an antioxidant include sulfite salt and ascorbic acid.

A medical composition according to the present embodiment can be produced according to a method commonly used in the field of drug manufacturing. The content of CFTR channel activator in a medical composition according to the present embodiment is not particularly limited as long as the CFTR channel activator can be administered in a specified dose (described later) with the medical composition, taking into account form and method of administration, etc.

A medical composition according to the present embodiment may be either orally administered in the form of, for example, a tablet, a capsule (soft capsule, micro capsule), a powder, a granule, or syrup, or non-orally administered in the form of, for example, injection, suppository, pellet, or drip infusion. A pharmaceutical composition according to the present invention can be toxic. However, by developing a less-toxic equivalent, it would be possible to administer the medical composition either orally or non-orally. As used herein, the term "non-orally" refers to form of administration, including intravenous injection or infusion, intramuscular injection or infusion, intra-abdominal injection or infusion, intrasternal injection or infusion, subcutaneous injection or infusion, and intra-articular injection or infusion.

A dose of a medical composition according to the present embodiment varies depending on such factors as type of subject, administration route, and symptoms. A person ordinary skill in the art will be able to suitably set an optimal condition according to these factors.

A medical composition according to the present embodiment can be administered in an administration route as suitably selected according to form of the drug. The method of administration is not particularly limited, and it can be made either internally or externally, or by injection. The injection may be made intravenously, intramuscularly, subcutaneously, or intradermally, for example.

A dose of a medical composition according to the present embodiment varies depending upon such factors as form of drug, method of administration, intended use, or age, weight, and symptoms of a patient exposed to the medical composition, and as such a dose is suitably set according to these factors. Generally, a daily dose of 5 to 400 μg/kg is preferable for adults in terms of an amount of active ingredient contained in the drug. However, since the dose varies depending on various conditions, a dose below or above this range may be sufficient or needed to exhibit the effects. Administration may be made either at once or in separate doses in a day, within a predetermined dose range. Further, a medical composition according to the present embodiment may be orally administered either directly or with any food or drinks in a patient's daily diet.

Taken together, a pharmaceutical composition according to the present invention at least includes a CFTR channel activator. The CFTR channel activator may comprise both PKA activator (or phosphodiesterase III inhibitor) and PKC activator. In other words, a pharmaceutical composition containing different kinds of PKA activators (or phosphodiesterase III inhibitor) and PKC activators also falls within a technical scope of the present invention.

Administration of a pharmaceutical composition according to the present invention can suppress cell death in ventricle muscle. Further, administration of a pharmaceutical composition according to the present invention can protect cardiac muscle in cardiac surgery and heart transplant. Further, administration of a pharmaceutical composition according to the present invention can treat diseases caused by necrosis.

(2) Evaluation Method of Cell Death Caused by Ischemia and Post-Ischemic Reperfusion (*in vitro* system)

The present invention provides a method for evaluating cell death caused by ischemia and post-ischemic reperfusion, in an *in vitro* cultured cell system.

An *in vitro* evaluation method of cell death according to the present invention includes: a step of preparing a cell culture to be evaluated; an ischemia-treatment step of culturing the cells in a glucose-free buffer (medium) under anaerobic conditions; a post-reperfusion-treatment step of culturing the ischemic cells in a glucose-containing medium under aerobic conditions; and a step of detecting cell death. An *in vitro* evaluation method of cell death according to the present invention exposes the tested cells to post-ischemic reperfusion. Thus, with an *in vitro* evaluation method of cell death according to the present invention, cell death can be evaluated concerning parameters such as viability, before and after a period of ischemia or post-ischemic reperfusion. Evaluation of reperfusion-induced cell death can then be made by simply comparing the results.

The type of cell evaluated by an *in vitro* evaluation method of cell death according to the present invention is not particularly limited. For example, myocardial cells, brain nerve cells, and brain glial cells can be suitably used. When myocardial cells are used, cell death can be evaluated that is caused by ischemia or post-ischemic reperfusion resulting from myocardial infarction. When brain nerve cells or brain glial cells are used, cell death can be evaluated that is caused by ischemia or post-ischemic reperfusion resulting from brain infarction, for example. The cells may be obtained from a commercially available cell culture, or cells obtained from primary culture may be used.

The type of buffer used in the ischemia-treatment step is not particularly limited as long as it is glucose-free. The composition of buffer is preferably 108 mM NaCl, 0.5 mM $MgCl_2$, 5.4 mM KCl, 1 mM $CaCl_2$, and 5 mM HEPES, which have been supplemented with $NaHCO_3$ and adjusted to pH 7.4.

In the ischemia-treatment step, cells are cultured under anaerobic conditions. As used herein, "under anaerobic conditions" is intended to mean an atmosphere with an oxygen concentration no greater than 1%, or more preferably an atmosphere that contains substantially no oxygen or no oxygen at all (oxygen concentration no greater than 0.01%). Therefore, it will be readily understood by a person ordinary skill in the art that "under anaerobic conditions" as used herein refers to conditions of cell culturing that is performed under carbon dioxide gas, nitrogen gas, and argon gas, and that a mixture of 95% argon gas and 5% carbon dioxide gas also falls within the meaning of "under anaerobic conditions." It will also be apparent to a person ordinary skill in that art that instruments and/or methods of cell incubation used in the invention can be suitably selected from those known in the art.

The incubation temperature in the ischemia-treatment step is selected according to temperature conditions that are suited for ordinary cell incubation. A temperature of 28°C to 40°C is preferable, and 37°C is most preferable.

The incubation time (process time) in the ischemia-treatment step should be sufficient to place the cells under ischemic conditions. For example, 6 to 10 hours is preferable, and 8 hours is most preferable.

The type of medium (buffer) used in the reperfusion-treatment step is not particularly limited as long as it includes glucose, and can be suitably selected from media known in the art. Preferable examples of such media include: M199 medium supplemented with 10% FBS; DMEM medium; RPMI medium; MEM medium; and EM medium.

The "aerobic conditions" in the reperfusion-treatment step refers to an atmosphere with an oxygen concentration no less than 5%, more preferably no less than 10%, and even more preferably no less than 15%. The "aerobic conditions" in the reperfusion-treatment step is most preferably a mixture of 95% air (about 20% oxygen) and 5% carbon dioxide gas, as commonly used in the art.

The incubation time in the reperfusion-treatment step is set such that the ischemic cells are at least perfused. A period of 72 to 120 hours is preferable, 84 to 108 hours is more preferable, and 96 hours is most preferable. The incubation temperature in the reperfusion-treatment step is set in the same manner as in the ischemia-treatment step. Preferably, the incubation is performed at 37°C for 96 hours.

In an *in vitro* evaluation method of cell death according to the present invention, the step of detecting cell death is not particularly limited. For example, detection of cell death can be made based on activity of mitochondrion dehydrogenase (MTT method or MTS method), fragmentation of intranuclear DNA (DNA ladder test), activity of cell death-causing intracellular protease (caspase), release of cytochrome c from mitochondrion into cytoplasm, or changes in morphology of the nucleus (for example, condensation or fragmentation of the nucleus).

Cell death accompanies a reduction in the activity of mitochondrion dehydrogenase, and therefore a reduced dehydrogenase activity can be regarded as an increased cell death rate, using activity of mitochondrion dehydrogenase as a measure.

Cell death caused by necrosis involves membrane injury in the cells, allowing the cells to be stained with propidium iodide (PI). By staining the nucleus of all cells with Hoechst 33342, the proportion of cells that have undergone necrotic cell death can be calculated.

Cell death caused by apoptosis involves fragmentation of intracellular DNA. Thus, by observing a state of intracellular DNA on ladders by electrophoresis for example, it is possible to readily determine whether the cell death is attributed to apoptotic cell death.

Further, since cell death caused by apoptosis is known to activate caspase (raise activity of the enzyme), an increase in enzyme activity can be regarded as an increased cell death rate in apoptotic cell death, using caspase activity as a measure.

Further, in apoptotic cell death induced by stimulation of mitochondria, the mitochondria release cytochrome c into the cytoplasm. This can be used as a measure of cell death. By measuring the quantity of cytochrome c released into the cytoplasm, it is possible to readily determine whether the cell death is attributed to apoptotic cell death.

Further, apoptotic cell death involves changes in morphology of the nucleus, such as condensation or fragmentation. This can also be used as a measure of cell death. By observing changes in morphology of the nucleus with an electron microscope for example, it is possible to readily determine whether the cell death is attributed to apoptotic cell death.

An *in vitro* evaluation method of cell death according to the present invention can advantageously be used for the screening of drugs intended to inhibit cell death caused by ischemia or post-ischemic reperfusion, and in particular drugs intended to suppress necrosis caused by ischemia or post-ischemic reperfusion. Specifically, by comparing results of comparison on cell death between a group that was exposed to a test drug before, during, or after the treatment (ischemia-treatment step or reperfusion-treatment step) and a group that was not exposed to the test drug in the treatment, it is possible to evaluate inhibitory effects of the drug against cell death caused by ischemia or post-ischemic reperfusion. This enables production of substances having inhibitory effects against cell death caused by ischemia or post-ischemic reperfusion.

(3) Evaluation Method of Cell Death Caused by Ischemia and Post-Ischemic Reperfusion (*in vivo* system)

The present invention provides a method for evaluating cell death induced by ischemia and post-ischemic reperfusion, in an *in vivo* system using animals.

Exposure of mice to ischemia and post-ischemic reperfusion is made according to common procedures known in the art. Specifically, the mice, anesthetized with ether and affixed to a surgical table are orally intubated. The animals are then connected to a ventilator and anesthetized with anesthetic gas. Respiration is managed and anesthetization is maintained. The left anterior descending coronary artery is ligated with suture to expose the mice to ischemia. After a specified period of ischemia, the ligature is severed to allow for reperfusion. After the episode of ischemia and reperfusion, the chest wall is closed and the mice, emerged from anesthesia, are kept according to common procedures.

However, ischemic and post-ischemic reperfusion treatment by this maneuver often fails *in vivo* due to a temperature drop in intrathoracic temperature and rectal temperature, and evaporation of body moisture during the open-heart procedure. Under these circumstances, the inventors of the present invention constructed a system for successfully inducing ischemia and post-ischemic reperfusion in mice.

A system for successfully inducing ischemia and post-ischemic reperfusion in mice according to the present invention includes a heat-insulating device for maintaining body temperature. Specifically, a system according to the present invention includes: a surgical table structured to circulate water that has been heated to 40°C with a constant temperature bath; and a device for maintaining heart temperature and preventing drying by supplying a heated physiological saline into the thoracic cavity and collecting it therefrom by suction, using a pump and a heater. Having this arrangement, the system successfully maintained intrathoracic and rectal temperatures of tested mice in a temperature range of 35.8°C to 36.4°C, and prevented evaporation of body moisture during the open-heart procedure.

A system according to the present invention preferably includes a ligature holder for ligating the left anterior descending coronary artery. The ligature holder preferably comprises an outer portion of an indwelling needle. With such a ligature holder, no accidental cutting of suture occurred, and the success rate of experiment improved greatly. When the ligature holder was made of an appropriately flexible material in a portion in contact with a ligated portion, and when the ligature was made with a small piece of cloth, 0.5 mm thick, between the ligature holder and the ligated portion, damage to blood vessels due to squeezed tissue was prevented and reperfusion was reliably induced.

Further, a system according to the present invention preferably includes a transjugular drug-infusion instrument. The transjugular drug-infusion instrument is preferably a needle with a length of 70 mm, an outer diameter 0.164 mm, and an inner diameter 0.1 mm. Such a transjugular drug-infusion instrument can be indwelled in jugular venous, enabling the drug to be safely infused over an extended time period, without causing damage to the jugular venous.

Briefly, an *in vivo* evaluation method of cell death according to the present invention uses a system for successfully inducing ischemia and post-ischemic reperfusion in mice according to the present invention. A system for successfully inducing ischemia and post-ischemic reperfusion in mice according to the present invention preferably includes a heat-insulating device for maintaining body temperature, and more preferably includes a ligature holder and a transjugular drug-infusion instrument. Further, in a system for successfully inducing ischemia and post-ischemic reperfusion *in vivo* in mice according to the present invention, it is preferable to perform transjugular administration of heparin sodium (150 units/kg (body weight) diluted and administered at a dose no greater than 100 μL before the start of ischemia, and 100 units/kg (body weight) diluted and administered at a dose no greater than 100 μL before the start of reperfusion), and protamine sulfate (10 mg was diluted with respect to 1000 units of heparin and administered at a dose no greater than 100 μL 2 minutes after the start of reperfusion), in order to ensure reperfusion.

A system according to the present invention can be used for the screening of CFTR activator *in vivo*. That is, in one aspect of the invention, a system according to the present invention is a system for screening CFTR activator *in vivo.* In a preferred embodiment, a system according to the present invention includes: a heat-insulating device for maintaining body temperature; a ligature holder; and a continuous drug-infusion instrument. In a system according to one embodiment of the present invention, the heat-insulating device is preferably a heated surgery table using a constant temperature bath. Further, in a system according to one embodiment of the present invention, the ligature holder may be an outer portion of an indwelling needle. Further, in a system according to the present invention, the continuous drug-infusion instrument is preferably a transjugular drug-infusion instrument. It is preferable that a system according to the present invention further includes an instrument for perfusing a heated physiological saline in the thoracic cavity, in order to maintain an external environment and temperature of the heart and prevent drying of the heart. In another embodiment of the present invention, a system according to the present invention includes means for administering a candidate factor of CFTR activator to a subject, either at the start of reperfusion or immediately before or after reperfusion, in order to screen CFTR activator *in vivo.* The administration means is preferably a continuous drug-infusion instrument. A system according to the present embodiment may further include means for measuring a size of infarct occurring in a subject.

The present invention also provides a method for screening CFTR activator *in vivo*, using a system according to the present invention. A screening method according to the present invention includes: a step of administering a candidate factor of CFTR activator to a subject, either at the start of reperfusion or immediately before or after reperfusion; and a step of measuring a size of infarct occurring in the subject. In a screening method according to the present invention, it is preferable that the administration step be performed transjugularly.

As described above, co-application of PKA activator and PKC activator imparts desirable effects as a CFTR activator. It is therefore preferable that a screening method according to the present invention include a step of comparing a size of infarct occurring in a subject with that that has occurred by co-application of PKA and PKC activators employed instead of the candidate factor in the administration step. Preferably, the PKA activator is milrinone. As used herein, the "candidate factor" includes CFTR activator that can activate CFTR alone.

With CFTR knockout mice, it is possible to determine whether the effects of candidate factor are CFTR dependent. As such, a screening method according to the present invention may include a step of comparing a size of infarct as measured in subject wild type mice with that measured in subject CFTR knockout mice.

The present invention is not limited to the description of the embodiments above, but may be altered by a skilled person within the scope of the claims. An embodiment based on a proper combination of technical means disclosed in different embodiments is encompassed in the technical scope of the present invention. The following will describe the present invention in more detail based on Examples. It should be appreciated however that the invention is not limited in any way by the following description.

[Examples]

In the Examples below, a primary culture system of neonatal rat ventricular myocytes was used for *in vitro* experiments. Mice were used for *in vivo* experiments. Through the *in vitro* and/or *in vivo* experiments, the inventors assessed whether:

(i) the protection mechanism against ischemic injury in myocardial cells afforded by co-activation of PKA and PKC *in vitro* is attributed to actions of CFTR;
(ii) actions of CFTR in the protection mechanism against myocardial cell ischemic injury are associated with protection against which type of cell death, necrosis or apoptosis;
(iii) administration of CFTR channel activator to mice *in vivo* inhibits cardiac muscle injury caused by ischemia or reperfusion, particularly necrotic cell death in myocardial cells;
(iv) administration of CFTR channel activator *in vivo* after the start of ischemia inhibits cardiac muscle injury caused by ischemia or reperfusion, particularly necrotic cell death in myocardial cells; and
(v) the expression level of CFTR *in vivo* influences the degree of ischemic injury in cardiac muscle.

[Example 1]

Previously, the inventors have reported that CFTR was expressed on rat ventricular myocytes, and that CFTR expression on the plasma membrane of the cells was transiently enhanced by induced ischemia (H. Uramoto, N. Takahashi, A. K. Dutta, R. Z. Sabirov, Y. Ando-Akatsuka, S. Morishima & Y. Okada. Ischemia-induced enhancement of CFTR expression on the plasma membrane in neonatal rat ventricular myocytes. Jpn J Physiol 53, 357-365 2003). The inventors have also reported that application of CFTR channel blockers during ischemia aggravated post-reperfusion cell recovery, and that application of PKA and PKC activators during ischemia enhanced post-reperfusion cell recovery (H. Uramoto, S. Morishima, Y. Ando-Akatsuka, Y. Nagasaki, N. Hagiwara and Y. Okada. A role of CFTR Cl- channel in the protection from ischemic injury in neonatal rat ventricular myocytes. Jpn. J. Physiol. 52, S35, 2002). Based on these findings, the inventors examined whether the improvement in ischemic injury afforded by CFTR channel activator was due to activation of CFTR channel, and, if so, how the CFTR channel was involved in ischemic injury in rat myocardial cells.

Primary cultures of ventricular myocytes were prepared from the hearts of neonatal rats by enzyme treatment. M199+10% FBS was used as culture medium. To reduce the number of contaminating non-myocardial cells, the medium was supplemented with 100 μM BrdU during incubation. The primary cell cultures of rat myocardial cells were subjected to ischemic treatment (removal of glucose and oxygen) and post-ischemic reperfusion according to the following procedures. The medium was replaced with a glucose-free solution, which contained 108 mM NaCl, 0.5 mM $MgCl_2$, 5.4 mM KCl, 1mM $CaCl_2$, and 5 mM HEPES (pH adjusted to 7.4 with $NaHCO_3$), and were incubated at 37°C for 8 hours under anaerobic conditions, which were created by replacing air with a gas mixture of 95% Ar and 5% $CO_2$. Immediately after incubation, the solution was replaced with a glucose-containing normal medium (M199+10% FBS), and then left in a $CO_2$ incubator (95% air (20% $O_2$), 5% $CO_2$, 37°C) for 96 hours (reperfusion: re-supply of glucose/reoxygenation). $Cl^-$ channel blocker (NPPB) was administered to the primary cell cultures of rat ventricular myocytes 1 hour prior to ischemic treatment, or at the start of ischemic treatment that lasted 8 hours. Cl- channel blocker (NPPB) was also administered over a time period of 18 hours after the start of post-ischemic reperfusion. Cell viability was assayed using MTT. To ensure accuracy in viable cell count, the assay was performed using cells obtained 96 hours after the start of post-ischemic reperfusion (Figure 1).

As previously reported, a significant reduction in cell viability due to CFTR channel blocker was observed only when CFTR channel blocker (50 μM NPPB) was administered during ischemic treatment, but not when administration was made 1 hour prior to ischemia or when it was continued for 18 hours from the start of post-ischemic reperfusion. These results suggest that the CFTR resistance to ischemia is exhibited during ischemia.

[Example 2]

Primary cultures of rat ventricular myocytes were prepared and cultured in the manner described in Example 1. The procedures of Example 1 were also used to expose the primary cultures of rat ventricular myocytes to ischemic treatment (removal of glucose and oxygen) and post-ischemic reperfusion.

During ischemic treatment, the primary cultures of rat ventricular myocytes were administered with CFTR channel activator (PKA activator dbcAMP (1mM) or PKC activator PMA (100 nM), or both (DP)), or CFTR channel activator + CFTR channel blocker (NPPB (50 μM), glibenclamide (500 μM), gemfibrozil (2 mM), or 9AC (2 mM)). Following administration, ischemia was induced for 8 hours, and, after 96 hours of subsequent reperfusion, cell viability was assayed using MTT (Figure 2). As shown in Figure 2, co-application of PKA activator dbcAMP and PKC activator PMA significantly increased cell viability, but this effect was completely abolished by the co-application of CFTR channel blocker. The result suggests that the effect observed in co-application of dbcAMP and PMA is due to activation of CFTR channel, and that the CFTR channel plays a role in the protection against ischemic injury in myocardial cells.

Application of dbcAMP or PMA alone could not produce the effect obtained by the co-application of dbcAMP and PMA, at least to the level where it can be called significant. This suggests that the effect is not due to the sole action of PMA, which is known to exhibit preconditioning-like effect.

[Example 3]

In the cardiac muscle, ischemia causes release of catecholamine into intercellular space. This results in an endogenous increase of cAMP. Phosphodiesterase III is an enzyme that inhibits degradation of cAMP, and it is known to be distributed over the heart. Milrinone is an inhibitor of this enzyme (R. M. Wallis, J. D. Corbin, S. H. Francis & P. Ellis. Tissue distribution of phosphodiesterase families and the effects of sildenafil on tissue cyclic nucleotides, platelet function, and the contractile responses of trabeculae carneae and aortic rings in vitro. Am J Cardiol 83, 3C-12C, 1999). It is also suggested that milrinone activates CFTR channel (B. R. Cobb, L. Fan, T. E. Kovacs, E. J. Sorscher & J. P. Clancy. Adenosine receptors and phosphodiesterase inhibitors stimulate Cl- secretion in Calu-3 cells. Am J Respir Cell Mol Biol 29, 410-418, 2003). The half-life of milrinone is 2 hours, and about 70% of milrinone in the blood circulates with protein by binding to it and is released into urine without being metabolized (L. A. Lehtonen S. Antila & P. J. Pentikainen. Pharmacokinetics and pharmacodynamics of intravenous inotropic agents. Clin Pharmacokinet 43, 187-203, 2004). In clinical applications, milrinone has been used for the treatment of acute heart failure for its potential to exhibit vasodilation effect and cardiotonic effect (Y. Ohizumi. Novel types of cardiotonic drugs. Folia Pharmacol Japon 100, 259-269, 1992).

The inventors of the present invention examined whether the phosphodiesterase inhibitor, milrinone, exhibits the inhibitory action against ischemic injury as a CFTR channel activator, as does the PKA activator dbcAMP.

Primary cultures of rat ventricular myocytes were prepared and cultured in the manner described in Example 1. The procedures of Example 1 were also used to expose the primary cultures of rat ventricular myocytes to ischemic treatment (removal of glucose and oxygen) and post-ischemic reperfusion.

During ischemic treatment, the primary cultures of rat ventricular myocytes were administered with CFTR channel activator (milrinone (0.73 μg, 1 ml), isoproterenol (10 nM), and PMA (100 nM) used together (MIP)), or CFTR channel activator + CFTR channel blocker (NPPB (50 μM), glibenclamide (500 μM), gemfibrozil (2 mM), or 9AC (2 mM)). Following administration, ischemia was induced for 8 hours, and, after 96 hours of subsequent reperfusion, cell viability was assayed using MTT (Figure 3).

As shown in Figure 3, cell viability was enhanced in cells that were subjected to co-application of milrinone and PMA, as in the case of dbcAMP. This effect was inhibited by all CFTR channel blockers. It was therefore found that milrinone had the CFTR channel-mediated protective action against ischemic injury in cardiac muscle.

[Example 4]

Primary cultures of rat ventricular myocytes were prepared and cultured in the manner described in Example 1. The procedures of Example 1 were also used to expose the primary cultures of rat ventricular myocytes to ischemic treatment (removal of glucose and oxygen).

In order to confirm whether the cell death that had occurred in the affected area of ischemia was due to ischemic injury, CFTR channel blocker (NPPB (50 μM))was administered to primary cultures of rat ventricular myocytes at the start of ischemic treatment which lasted 8 hours. Immediately after ischemia, cells were double stained to examine a rate of necrotic cell death, using Hoechst 33342 and propidium iodide (PI) according to a method known in the art. Hoechst 33342 stains the nucleus of all cells, and propidium iodide (PI) stains only dead cells with necrosis-induced membrane injury.

After 8 hours of ischemia, 44.4% of cells were stained by PI in a group that had no reagent, whereas 90.3% of cells were PI stained in a group administered with NPPB (Figure 4).

The result suggests that a reduction in cell viability observed by MTT 96 hours after reperfusion is indeed due to necrosis.

[Example 5]

Involvement of apoptosis in the ischemic injury involving CFTR channel was examined.

Primary cultures of rat ventricular myocytes were prepared and cultured in the manner described in Example 1. The procedures of Example 1 were also used to expose the primary cultures of rat ventricular myocytes to ischemic treatment (removal of glucose and oxygen) and post-ischemic reperfusion.

The primary cultures of rat ventricular myocytes were administered with CFTR channel activator (PKA activator dbcAMP (1mM) + PKC activator PMA (100 nM)), or CFTR channel blocker (NPPB (50 μM)). Administration was made at the start of ischemic treatment, which lasted 8 hours. Fragmentation of nuclear DNA in the cells was assayed immediately after ischemia or 96 hours after post-ischemic reperfusion (Figure 5). Specifically, DNA fragmentation was assayed using a method (DNA fragmentation detecting method (Cell Death Detection ELIZA kit$^{PLUS}$ (Roche Diagnostics), which specifically detects mononucleotides and/or oligonucleotides that are characteristics of apoptotic cells. The result of measurement was obtained as enrichment factor, which was calculated according to the following formula.

Enrichment factor (rate of nuclear DNA fragmentation) = absorbance of sample (dead cells)/absorbance of control (untreated cells)

As shown in Figure 5, the level of DNA fragmentation (score of 1 for non-ischemic cells) immediately after ischemia was the same between non-ischemic cells and any of the non-administered group and the groups administered with CFTR channel blocker and CFTR channel activator. The result suggests that the cell death immediately after ischemia as observed by NPPB administration does not involve apoptosis, i.e., CFTR has the protective action against necrosis, not apoptosis.

Ninety-six hours after reperfusion, the level of DNA fragmentation increased three times; however, there was no significant difference between the non-administered group and the groups administered with CFTR channel blocker and CFTR channel activator. The result suggests that CFTR channel is not involved in apoptosis after reperfusion.

[Example 6]

Male mice (C57BL/6J, CLEA Japan, Inc.), 9 weeks of age, were used for *in vivo* experiment. The mice were anesthetized with ether. The animals were affixed to a surgery table and orally intubated, and then connected to a ventilator and anesthetized with a mixed gas of oxygen (20%) and nitrous oxide plus 1% sevoflurane. Respiration was managed and anesthetization was maintained. The left anterior descending coronary artery was ligated with suture. Thirty minutes after occlusion, the ligature was severed to allow for reperfusion. Administration of drugs was made transjugularly. After an episode of ischemia and reperfusion, a piece of suture was left in the thoracic cavity and the chest wall was closed. The mice, emerged from anesthesia, were kept according to common procedures. After two days, the mice were anesthetized with ether and the chest wall was opened. The animals were then religated and evans blue was infused into the left ventricle through the apex cordis. The heart was immediately excised and cut in 1-mm-thick cross sections. Viable regions of the heart sections were stained with triphenyltetrazolium chloride (TTC) to determine a degree of ischemia-induced cardiac muscle injury as a ratio of infarct area (unstained with TTC) to ischemic area.

For the *in vivo* experiment, the following systems were used.

(a) A heat-insulating system for maintaining body temperature (Figure 6), including:

(i) a surgery table: structured to circulate 40°C water using a constant temperature bath; and
(ii) a system for maintaining heart temperature and preventing drying of the heart: sending heated physiological saline into the thoracic cavity using a pump and a heater, and collecting it by suction.

With (i) and (ii), it was possible to maintain intrathoracic temperature and rectal temperature in a range of 35.8°C to 35.4°C, and prevent evaporation of body moisture during the open-heart procedure, thereby enabling the experiment.

(b) A ligature holder: The left anterior descending coronary artery is ligated using an outer portion of an indwelling needle, as shown in Figure 7. With this tool, there was no accidental cutting of the suture, and the success rate of experiment improved. When the ligature was made with a small piece of cloth, 0.5 mm thick, damage to the ligated tissues was suppressed and reperfusion was reliably induced.

(c) A transjugular drug-infusion system: A MicroFil™ (World Precision Instruments, INC: a needle with a length of 70 mm, an outer diameter 0.164 mm, and an inner diameter 0.1 mm) was used, as shown in Figure 8. MicroFilm™ can be indwelled in jugular venous, enabling the drug to be safely infused over an extended time period, without causing damage to the jugular venous.

[Example 7]

Generally, *in vitro* experiment uses dibutyryl cyclic AMP (dbcAMP), forskolin, and isoproterenol as PKA activators, for example. However, there are potential adverse effects in using these drugs in *in vivo* experiment. The PKA activator, milrinone, is considered to be suitable for *in vivo* experiment because it activates CFTR channel and has been used in clinical applications.

*In vivo* experiment was conducted using mice, according to the procedures of Example 6. As shown in Figure 9A, a physiological saline, containing both milrinone and PMA, was administered in one shot to C57BL/ 6J mice at the concentrations shown in Figure 9B, 10 minutes prior to the start of ischemia. Administration was continued until the end of ischemia at such a rate that 1/3 of the amount used in one shot was infused per hour.

A significant reduction in infarct size was observed by this protocol. This suggests that CFTR activator exhibits the suppressing action against ischemic injury in cardiac muscle also *in vivo*.

Further, assessment was made regarding administration time of the drugs. The procedure of Example 6 was used for the *in vivo* experiment using mice. The drugs were administered at different times as shown in Figure 10A, and a proportion of an infarct region with respect to the ischemic region was examined (Figure 10B). In 30 minutes of ischemia, the administration of the drugs in one shot at the start of reperfusion exhibited substantially the same effect of reducing infarct size as in the continuous administration, which was started prior to ischemia and lasted until the start of reperfusion.

Further, using CFTR knockout mice ($Cftr^{tm1Unc}$), assessment was made as to whether the inhibiting effects of CFTR activator on ischemic injury was due to the action of CFTR. It is noted here that -/- mice could not be used in the experiment due to a significantly low survival rate. In the experiment, +/- (heterozygous) mice or +/+ (WT) mice were used. The procedure of Example 6 was used for the *in vivo* experiment using mice. After an episode of ischemia and reperfusion as shown in Figure 11A, the size of resulting infarct was measured. In the heterozygous mice with low-level CFTR expression, the size of infarct induced by ischemia and reperfusion was comparatively greater than that observed in WT mice, and there was aggravation of ischemic injury.

The present invention shows for the first time that activation of CFTR channel during ischemia, and at the start of, or immediately before or after reperfusion that follows a relatively short period of ischemia not causing irreversible injury, is effective for the protection against various kinds of ischemic injury induced by necrosis in myocardial cells.

Specifically, experiments were conducted based on a ischemia/reperfusion model in which cultured ventricular myocytes were re-administered with glucose and supplied with oxygen following glucose removal and oxygen deprivation. In the experiments, assessments were made in regard to: cell viability measured using MTT; apoptosis as determined by DNA fragmentation using ELIZA detection method; and necrosis as determined by double staining using Hoechst 33342 and propidium iodide (PI). As a result, there was established an *in vitro* assay for determining effectiveness of treatment/protective drugs against myocardial cell death caused by ischemic injury. An *in vivo* ischemia/reperfusion system (including a continuous drug-administration system) was also established. This led to the finding that ischemia-induced cell death could be suppressed by administration of CFTR channel activator before or during ischemia, or at the start of reperfusion.

There has been a report that activation of CFTR channel by β-adrenalin stimulation brings about a regulatory volume decrease (RVD) in myocardial cells that have swelled by osmosis (Z. Wang, T. Mituiye, S. A. Rees & A. Noma. Regulatory volume decrease of cardiac myocytes induced by β-adrenergic activation of the C1- channel in guinea pig. J Gen Physiol 10, 73-82, 1997). It is therefore believed that the protective mechanism against necrosis according to the present invention is due to the suppressing action of CFTR against abnormal cell swelling that causes necrosis in ischemic injury, the effect of which action is to restore the cell volume to original level.

The embodiments and concrete examples of implementation discussed in the foregoing detailed explanation serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.

INDUSTRIAL APPLICABILITY

Having the foregoing arrangement, a pharmaceutical composition according to the present invention can suppress cell death in ventricle muscle. A pharmaceutical composition according to the present invention can also protect cardiac muscle during cardiac surgery. Further, a pharmaceutical composition according to the present invention can be used for prevention of disease induced by necrosis.

Having the foregoing arrangement, an *in vivo* system for ischemia and post-ischemic reperfusion according to the present invention can maintain intrathoracic temperature and rectal temperature of test mice within a range of 35.8°C to 36.4°C, and prevent evaporation of body moisture during open-heart procedures. This enables an experiment to be successfully performed.

The present invention provides novel protection against ischemic cardiac muscle injury, targeting at CFTR which has not been considered as a protective factor of ischemic injury. The present invention therefore offers improvements when applied to myocardial protection methods in cardiac surgery and heart transplant where ischemic injury may be a problem. The present invention is also effective in administration during reperfusion, and is therefore applicable to administration after the onset of ischemia, or more specifically, to medical therapy.

## Claims

1. A system for screening a CFTR activator *in vivo,* comprising: a heat-insulating device for maintaining body temperature; a ligature holder; and a continuous drug-infusion instrument.

2. A system according to claim 1, wherein the heat-insulating device comprises a heated surgery table using a constant temperature bath.

3. A system according to claim 1, wherein the ligature holder comprises an outer portion of an indwelling needle.

4. A system according to claim 1 wherein the continuous drug-infusion instrument comprises a transjugular infusion instrument.

5. A system according to claim 1, further comprising an instrument for perfusing a heated physiological saline in the thoracic cavity so as to maintain an external environment and temperature of the heart and prevent drying of the heart.

6. A method for screening a CFTR activator *in vivo* using a system of claims 1 to 5, the method comprising the steps of:

   administering a candidate factor of CFTR activator to a subject, either at a start of reperfusion or immediately before or after reperfusion; and
   measuring a size of infarct occurring in the subject.

7. A method according to claim 6, wherein the administration step is performed transjugularly.

8. A method according to claim 6, comprising the step of comparing the size of infarct occurring in the subject with a size of infarct that has occurred by co-application of a PKA activator and a PKC activator, used instead of the candidate factor in the administration step.

9. A method according to claim 8, wherein the PKA activator is milrinone.

10. A method according to claim 6, comprising the step of comparing the size of infarct occurring in the subject with a size of infarct that has occurred by application of a CFTR activator, used alone instead of the candidate factor in the administration step.

11. A method according to claim 6, comprising the step of comparing a size of infarct measured in subject wild type mice with a size of infarct measured in subject CFTR knockout mice.

12. A pharmaceutical composition for suppressing cell death in ventricle muscle, comprising a CFTR channel activator.

13. A pharmaceutical composition according to claim 12, wherein the cell death comprises necrosis.

14. A pharmaceutical composition according to claim 12, wherein the cell death is caused by ischemia or post-ischemic reperfusion.

15. A pharmaceutical composition according to claim 12, wherein the CFTR channel activator comprises a mixture of PKA activator and PKC activator.

16. A pharmaceutical composition according to claim 15, wherein the PKA activator is dibutyryl cyclic AMP.

17. A pharmaceutical composition according to claim 15, wherein the PKC activator is phorbol 12-myristate 13-acetate.

**18.** A pharmaceutical composition according to claim 12, wherein the CFTR channel activator includes phosphodiesterase III inhibitor and PKC activator.

**19.** A pharmaceutical composition according to claim 18, wherein the phosphodiesterase III inhibitor is 1,6-dihydro-2-methyl-6-oxo[3,4'-bipyridine]-5-carbonitrile.

**20.** A pharmaceutical composition according to claim 12, wherein the cell death is induced by abnormalities that are caused during ischemia in myocardial tissues.

**21.** A pharmaceutical composition according to claim 12, wherein the cell death accompanies a reduction in activity of mitochondrion dehydrogenase.

**22.** A pharmaceutical composition according to claim 12, which is used to suppress necrotic cell death in a primary culture of ventricular myocytes.

**23.** A pharmaceutical composition according to claim 12, which reduces an infarct area in ventricle muscle.

**24.** A pharmaceutical composition for protecting cardiac muscle in cardiac surgery and heart transplant, comprising a CFTR channel activator.

**25.** A pharmaceutical composition according to claim 24, which is used in cardiac surgery or heart transplant of a necrotic heart.

**26.** A pharmaceutical composition according to claim 24, wherein the CFTR channel activator comprises a mixture of PKA activator and PKC activator.

**27.** A pharmaceutical composition according to claim 26, wherein the PKA activator is dibutyryl cyclic AMP.

**28.** A pharmaceutical composition according to claim 26, wherein the PKC activator is phorbol 12-myristate 13-acetate.

**29.** A pharmaceutical composition according to claim 24, wherein the CFTR channel activator includes phosphodiesterase III inhibitor and PKC activator.

**30.** A pharmaceutical composition according to claim 29, wherein the phosphodiesterase III inhibitor is 1,6-dihydro-2-methyl-6-oxo[3,4'-bipyridine]-5-carbonitrile.

**31.** A pharmaceutical composition for preventing diseases induced by necrosis, comprising a CFTR channel activator.

**32.** A pharmaceutical composition according to claim 31, wherein the CFTR channel activator comprises a mixture of PKA activator and PKC activator.

**33.** A pharmaceutical composition according to claim 32, wherein the PKA activator is dibutyryl cyclic AMP.

**34.** A pharmaceutical composition according to claim 32, wherein the PKC activator is phorbol 12-myristate 13-acetate.

**35.** A pharmaceutical composition according to claim 31, wherein the CFTR channel activator includes phosphodiesterase III inhibitor and PKC activator.

**36.** A pharmaceutical composition according to claim 35, wherein the phosphodiesterase III inhibitor is 1,6-dihydro-2-methyl-6-oxo[3,4'-bipyridine]-5-carbonitrile.

**37.** A method for suppressing cell death in ventricle muscle, comprising the step of administering a CFTR channel activator.

**38.** A method for protecting cardiac muscle in cardiac surgery and heart transplant, comprising the step of administering a CFTR channel activator.

**39.** A method for preventing diseases induced by necrosis, comprising the step of administering a CFTR channel activator.

**40.** A system for inducing ischemia and post-ischemic reperfusion *in vivo,* comprising a heat-insulating device for maintaining body temperature.

**41.** A system for inducing ischemia and post-ischemic reperfusion according to claim 40, further comprising a ligature holder and a continuous drug-infusion instrument.

**42.** A system for inducing ischemia and post-ischemic reperfusion according to claim 40, wherein the heat-insulating device comprises a heated surgery table using a constant temperature bath.

**43.** A system for inducing ischemia and post-ischemic reperfusion according to claim 40, further comprising an instrument for perfusing a heated physiological saline in the thoracic cavity so as to maintain an extracellular environment and temperature of the heart and prevent drying of the heart.

**44.** A system for inducing ischemia and post-ischemic reperfusion according to claim 40, wherein the ligature holder comprises an outer portion of an indwelling needle.

FIG. 1

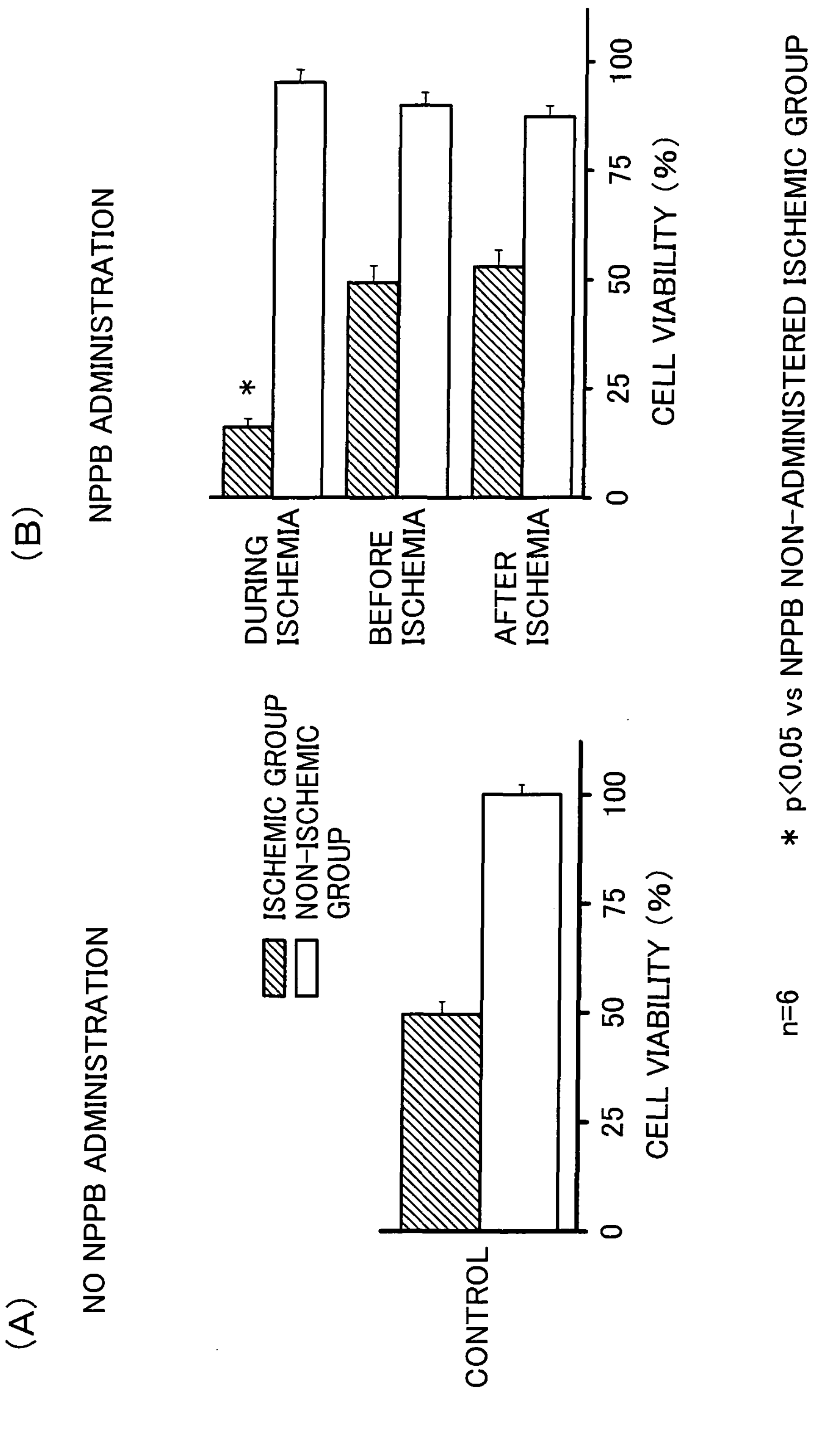
(A)
NO NPPB ADMINISTRATION
ISCHEMIC GROUP
NON-ISCHEMIC GROUP
CONTROL
0
25
50
75
100
CELL VIABILITY (%)
n=6
(B)
NPPB ADMINISTRATION
DURING ISCHEMIA
BEFORE ISCHEMIA
AFTER ISCHEMIA
*
0
25
50
75
100
CELL VIABILITY (%)
* p<0.05 vs NPPB NON-ADMINISTERED ISCHEMIC GROUP

FIG. 2

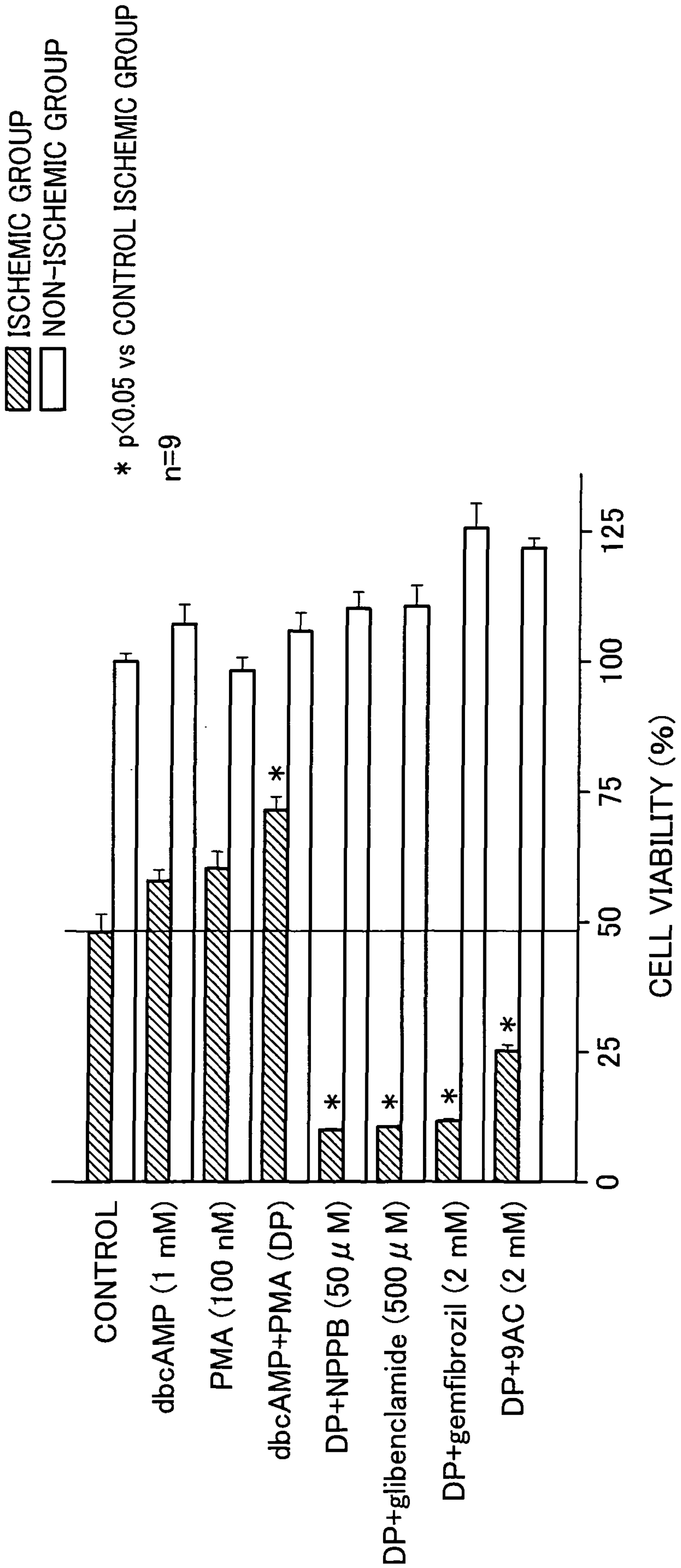
ISCHEMIC GROUP
NON-ISCHEMIC GROUP
* p<0.05 vs CONTROL ISCHEMIC GROUP
n=9
CONTROL
dbcAMP (1 mM)
PMA (100 nM)
dbcAMP+PMA (DP)
DP+NPPB (50 μM)
DP+glibenclamide (500 μM)
DP+gemfibrozil (2 mM)
DP+9AC (2 mM)
0
25
50
75
100
125
CELL VIABILITY (%)

FIG. 3

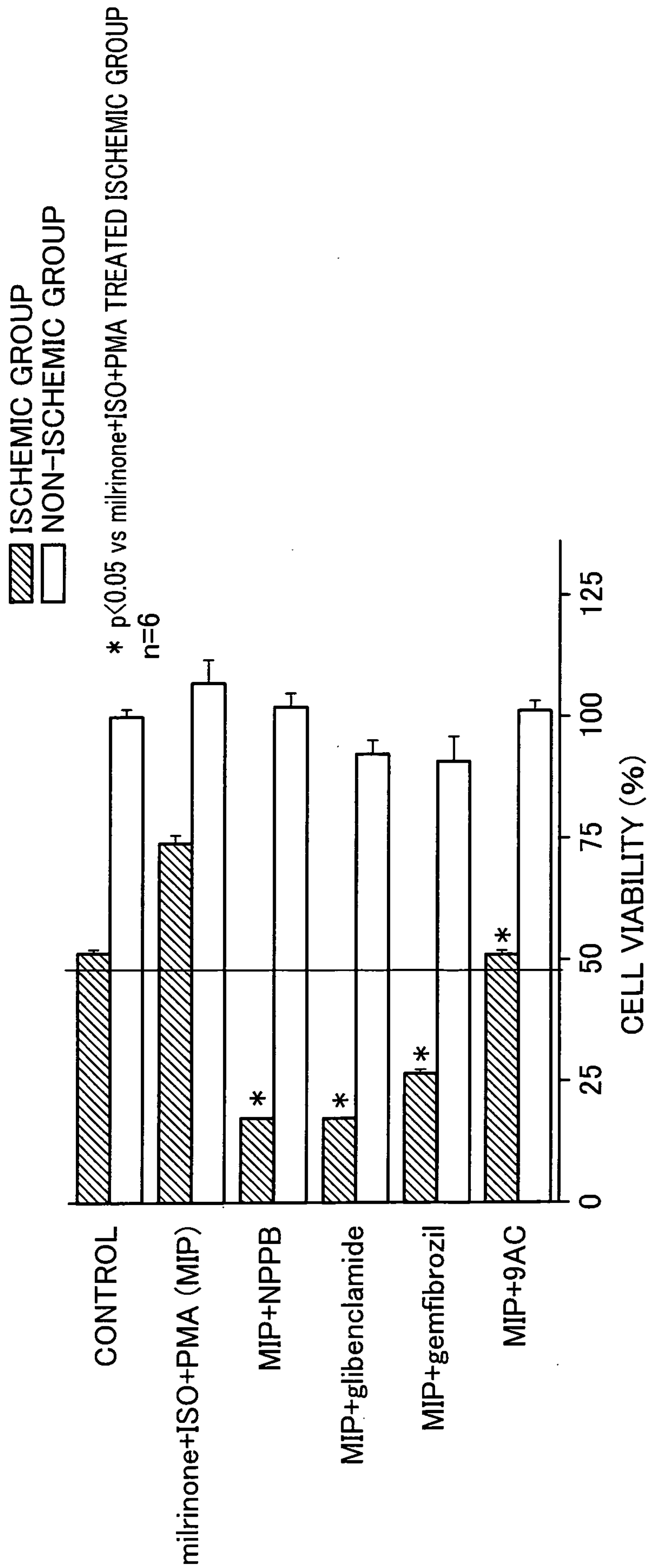
ISCHEMIC GROUP
NON-ISCHEMIC GROUP
* p<0.05 vs milrinone+ISO+PMA TREATED ISCHEMIC GROUP
n=6
CONTROL
milrinone+ISO+PMA (MIP)
MIP+NPPB
MIP+glibenclamide
MIP+gemfibrozil
MIP+9AC
0
25
50
75
100
125
CELL VIABILITY (%)

FIG. 4

(A)

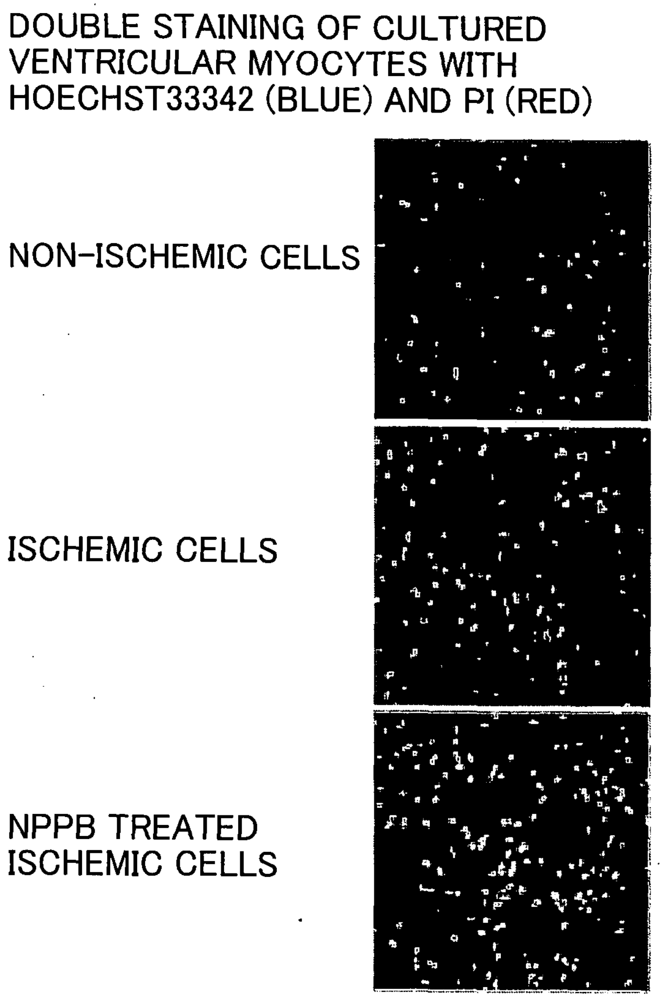
DOUBLE STAINING OF CULTURED VENTRICULAR MYOCYTES WITH HOECHST33342 (BLUE) AND PI (RED)
NON-ISCHEMIC CELLS
ISCHEMIC CELLS
NPPB TREATED ISCHEMIC CELLS

(B)

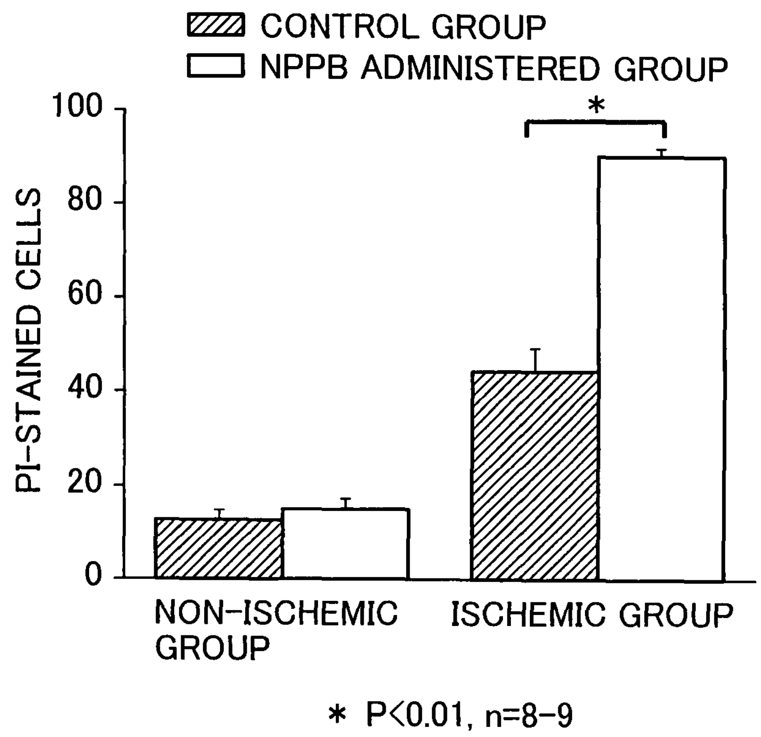
CONTROL GROUP
NPPB ADMINISTERED GROUP
PI-STAINED CELLS
100
80
60
40
20
0
*
NON-ISCHEMIC GROUP
ISCHEMIC GROUP
* P<0.01, n=8-9

FIG. 5

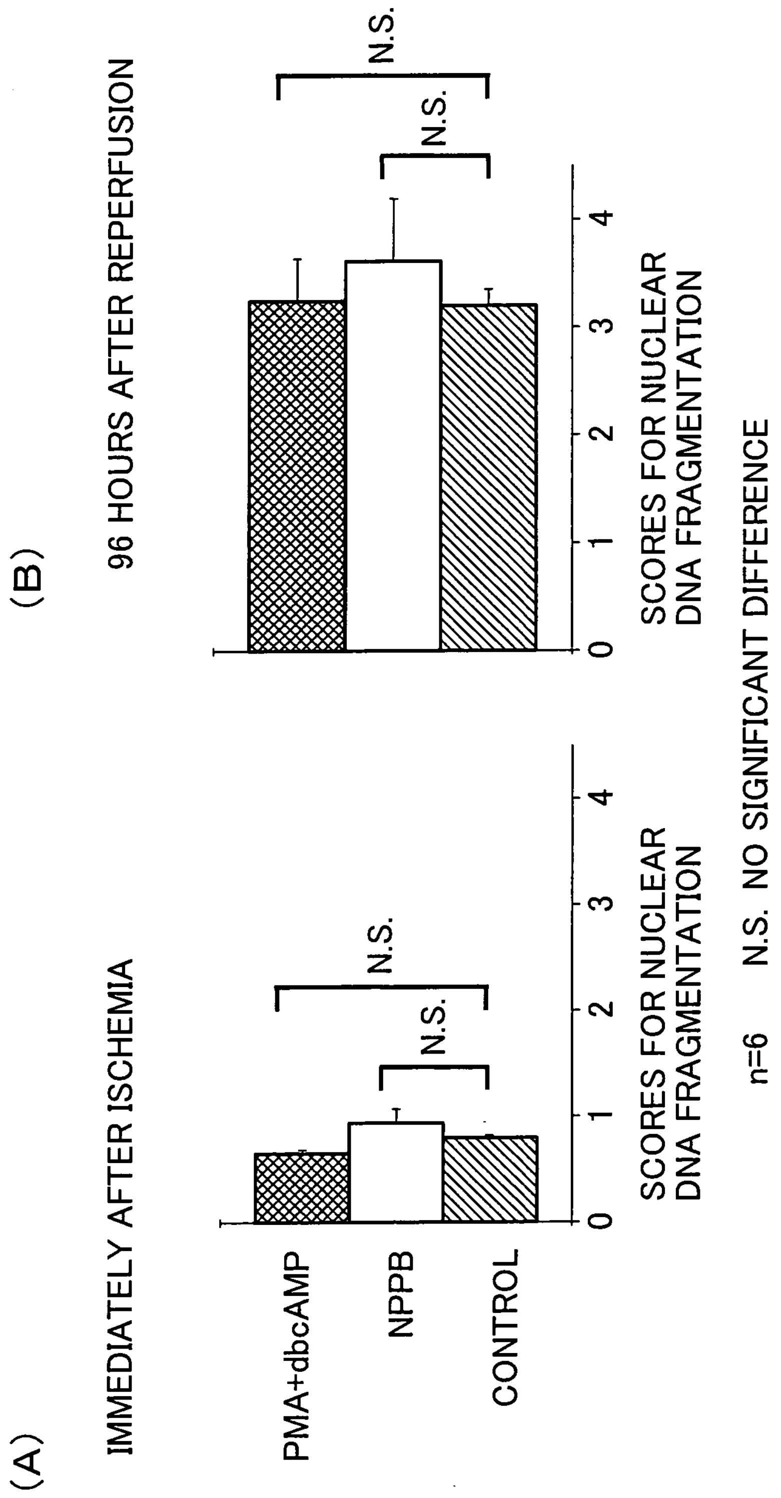
(A)
IMMEDIATELY AFTER ISCHEMIA
PMA+dbcAMP
NPPB
CONTROL
N.S.
N.S.
0
1
2
3
4
SCORES FOR NUCLEAR DNA FRAGMENTATION
(B)
96 HOURS AFTER REPERFUSION
N.S.
N.S.
0
1
2
3
4
SCORES FOR NUCLEAR DNA FRAGMENTATION
n=6
N.S. NO SIGNIFICANT DIFFERENCE

FIG. 6

(A)

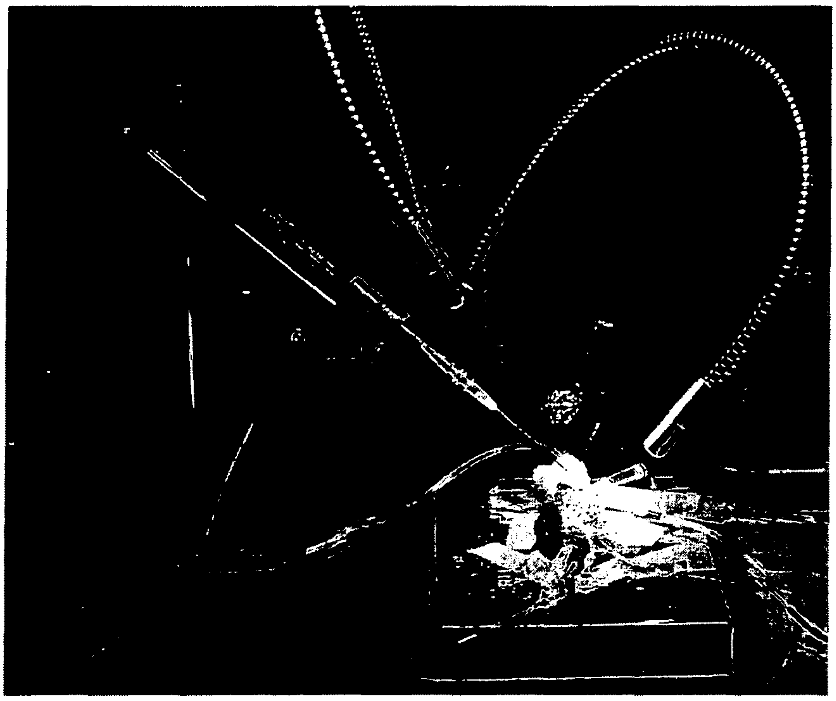

(B)

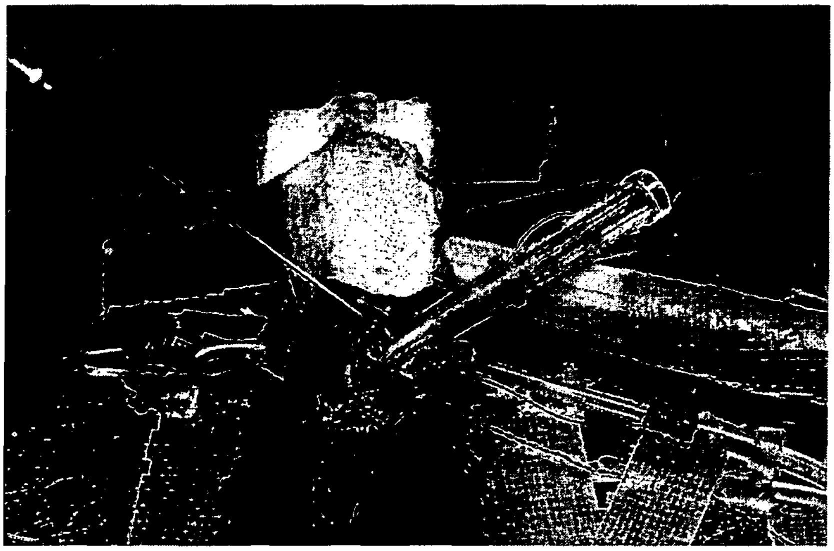

FIG. 7

(A)

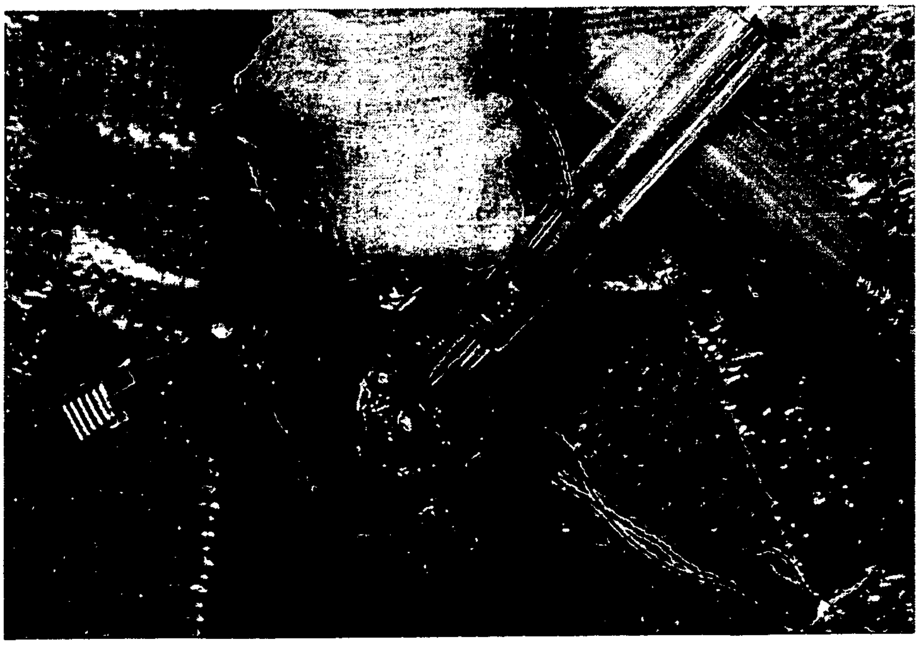

(B)

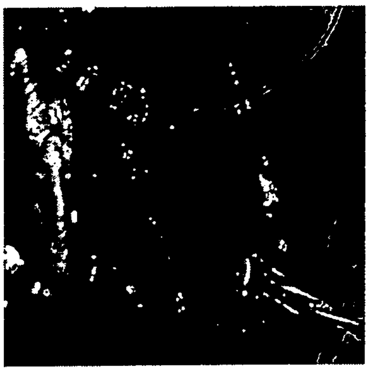

(C)

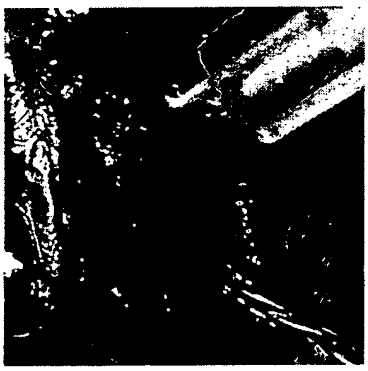

FIG. 8

(A) (B)

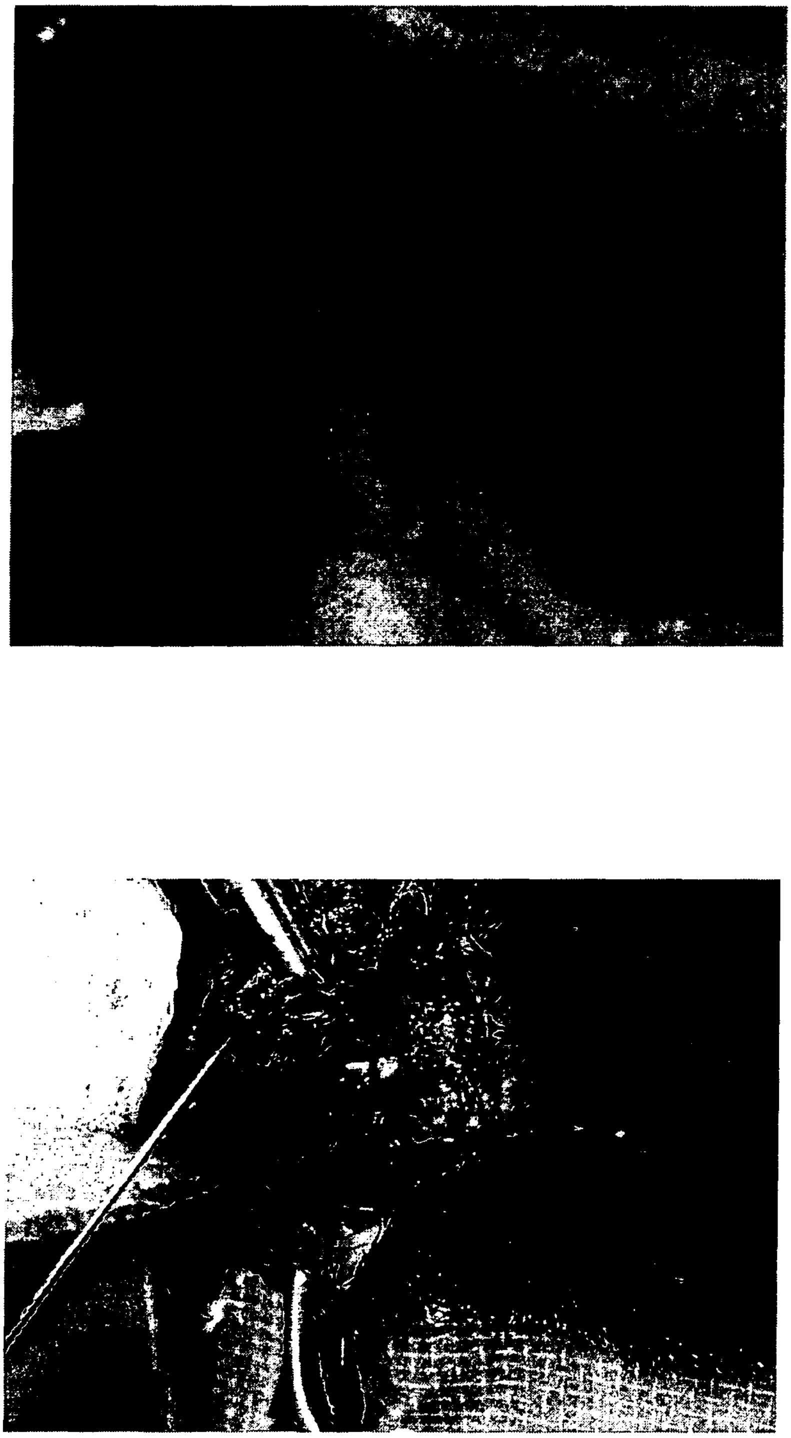

FIG. 9

(A)

(B)

(C)

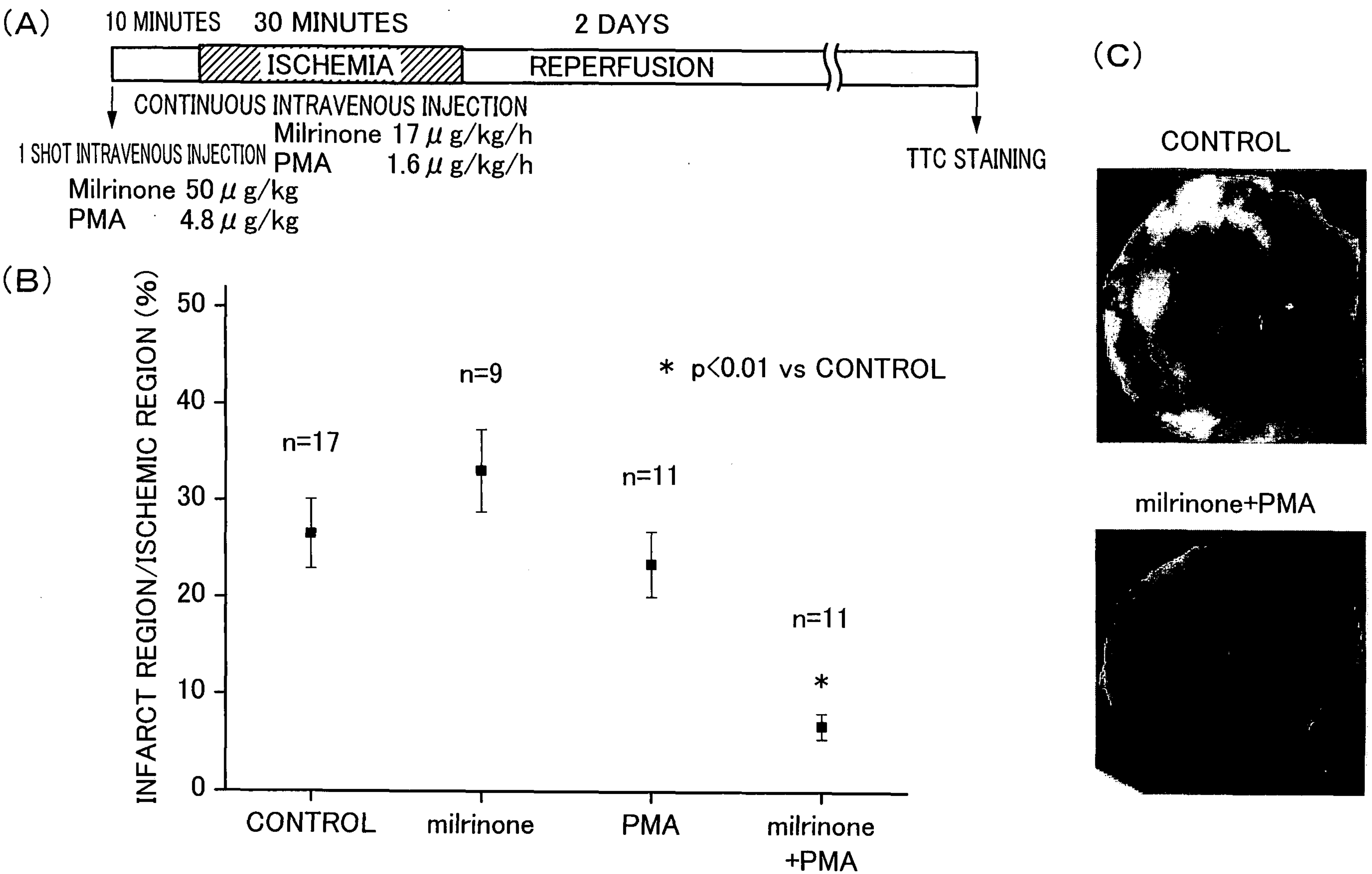
10 MINUTES
30 MINUTES
2 DAYS
ISCHEMIA
REPERFUSION
CONTINUOUS INTRAVENOUS INJECTION
Milrinone 17 μ g/kg/h
PMA 1.6 μ g/kg/h
1 SHOT INTRAVENOUS INJECTION
Milrinone 50 μ g/kg
PMA 4.8 μ g/kg
TTC STAINING
INFARCT REGION/ISCHEMIC REGION (%)
50
40
30
20
10
0
n=17
n=9
n=11
n=11
*
* p<0.01 vs CONTROL
CONTROL
milrinone
PMA
milrinone +PMA
CONTROL
milrinone+PMA

FIG. 10

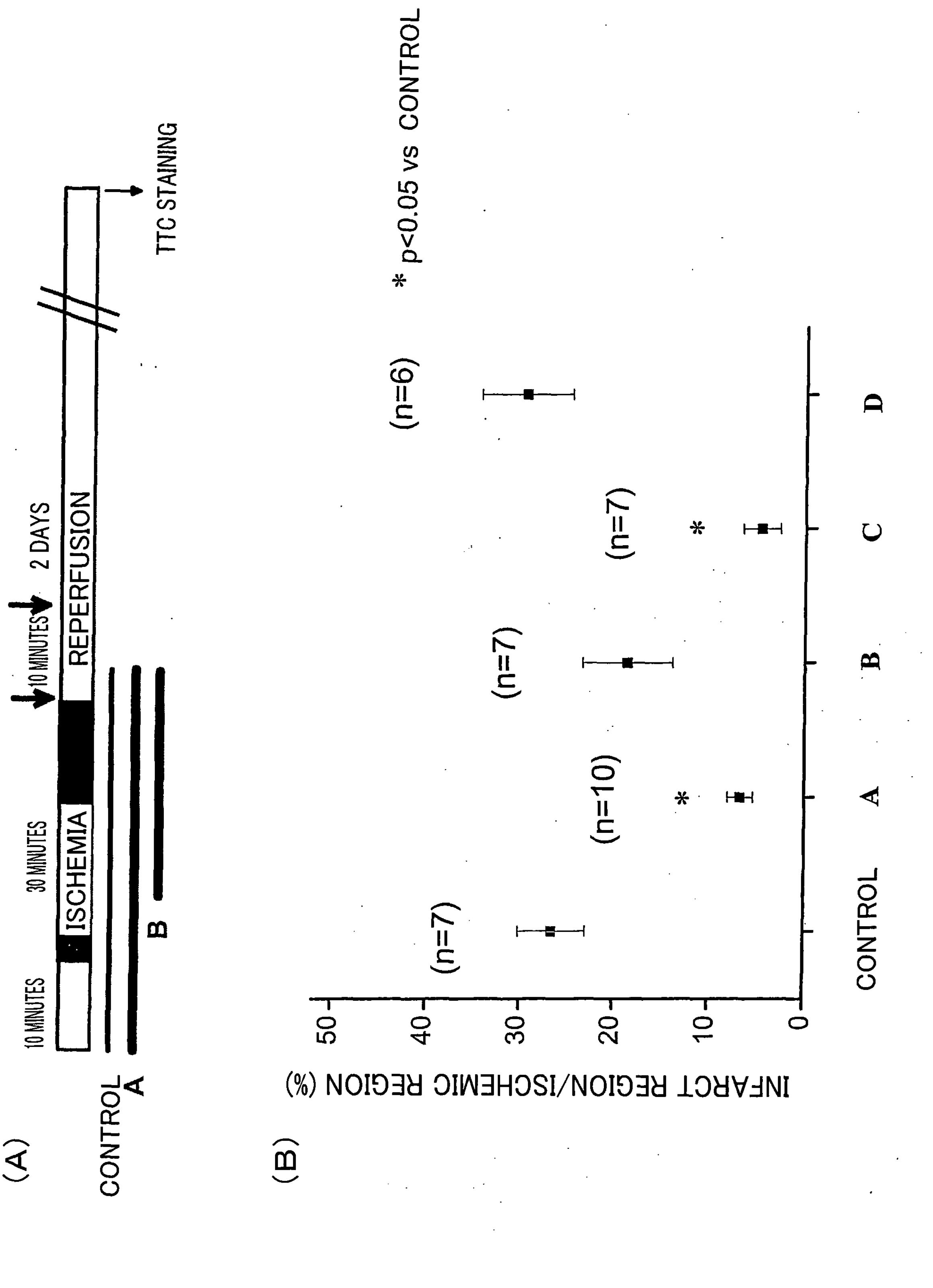
(A)
CONTROL
A
B
10 MINUTES
30 MINUTES
ISCHEMIA
C
D
10 MINUTES
REPERFUSION
2 DAYS
TTC STAINING
(B)
INFARCT REGION/ISCHEMIC REGION (%)
50
40
30
20
10
0
(n=7)
(n=10)
(n=7)
(n=7)
(n=6)
*
*
CONTROL
A
B
C
D
* p<0.05 vs CONTROL

FIG. 11

(A)

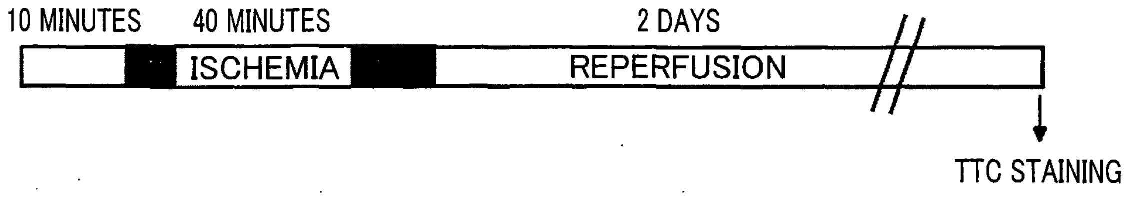
10 MINUTES
40 MINUTES
2 DAYS
ISCHEMIA
REPERFUSION
TTC STAINING

(B)

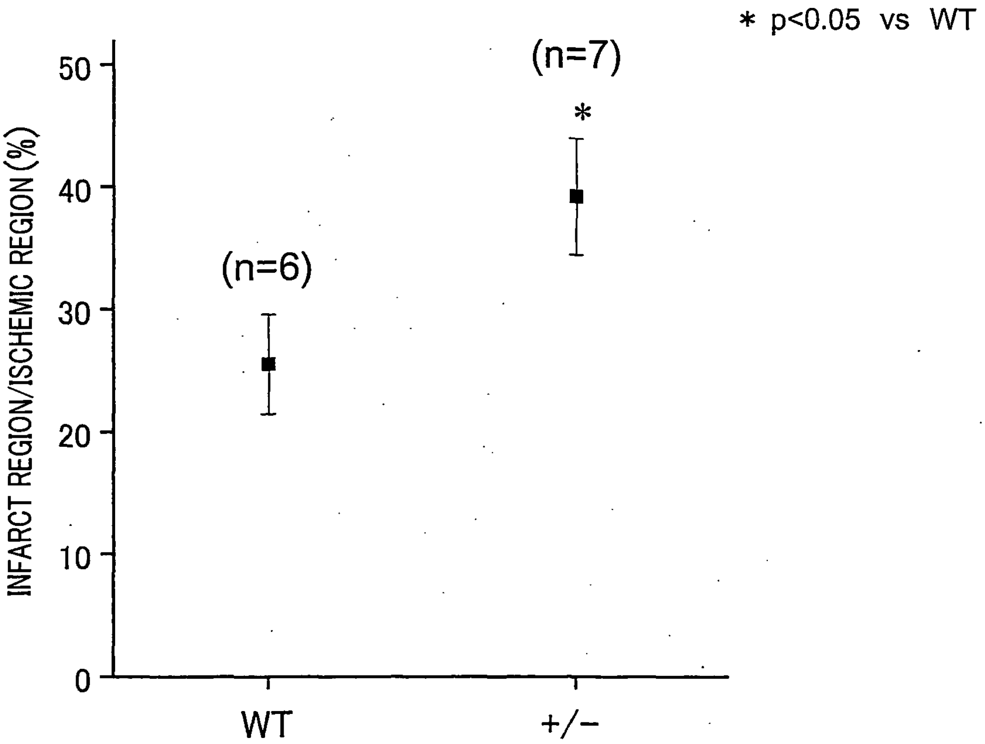
* p<0.05 vs WT
INFARCT REGION/ISCHEMIC REGION (%)
50
40
30
20
10
0
(n=6)
(n=7)
*
WT
+/−

**INTERNATIONAL SEARCH REPORT**

International application No.
PCT/JP2005/017494

A. CLASSIFICATION OF SUBJECT MATTER
***A61K45/00*** (2006.01), ***A61K31/23*** (2006.01), ***A61K31/4545*** (2006.01), ***A61K31/7076*** (2006.01), ***A61P9/00*** (2006.01), ***A61P9/10*** (2006.01), ***A61P43/00*** (2006.01), ***C12N5/06*** (2006.01), ***G01N33/15*** (2006.01), ***A61G13/00*** (2006.01)
According to International Patent Classification (IPC) or to both national classification and IPC

B. FIELDS SEARCHED
Minimum documentation searched (classification system followed by classification symbols)
***A61K45/00*** (2006.01), ***A61K31/23*** (2006.01), ***A61K31/4545*** (2006.01), ***A61K31/7076*** (2006.01), ***A61P9/00*** (2006.01), ***A61P9/10*** (2006.01), ***A61P43/00*** (2006.01), ***C12N5/06*** (2006.01), ***G01N33/15*** (2006.01), ***A61G13/00*** (2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
WPI, BIOSIS(STN), CAplus(STN), EMBASE(STN), MEDLINE(STN), JMEDPlus(JOIS)

C. DOCUMENTS CONSIDERED TO BE RELEVANT

| Category* | Citation of document, with indication, where appropriate, of the relevant passages | Relevant to claim No. |
|---|---|---|
| X<br>Y | de TASSIGNY, Alezxandra d'Anglemont et al., Hypo-osmotic stress inhibits doxorubicin-induced apoptosis via a protein kinase A-dependent mechanism in cardiomyocytes, Clinical and Experimental Pharmacology and Physiology, July 2004, Vol.31, No.7, pages 438 to 443, Abstract | 12-14<br>15-17,21,22, 26,27 |
| X<br>Y | JUNG, Yi-Sook et al., Essential role of PKC-epsilon in the protective effect of high glucose against hypoxic injury in cardiac myoblast., FASEB Journal, 07 March, 2001 (07.03.01), Vol.15, No.4, p.A84.. full text | 12-14<br>15-17,21,22, 26-30 |

☒ Further documents are listed in the continuation of Box C.　　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 November, 2005 (24.11.05) | 06 December, 2005 (06.12.05) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2005/017494

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | YAO, Zhenhai et al., Signal transduction of flumazenil-induced preconditioning in myocytes, American Journal of Physiology, 2001, Vol.280, No.3, Pt.2, pages H1249 to H1255, Abstract | 12-14<br>15-22,26-30 |
| X<br>Y | ENDOH, M., Changes in intracellular Ca2+ mobilization and Ca2+ sensitization as mechanisms of action of physiological interventions and inotropic agants in intact myocardial cells, Jpn. Heart J., 1998, Vol.39, No.1, pages 1 to 44, Abstract, page 6, line 18 to page 8, line 12; page 13, line 19 to page 14, line 24 | 12-14<br>15-22,26-30, 35,36 |
| X<br>Y | SANADA, S. et al., Cardioprotective effect afforded by transient exposure to phosphodiesterase III inhibitors: the role of protein kinase A and p38 mitogen-activated protein kinase, Circularion, 2001, Vol.104, No.6, pp.705-10. Abstract | 12-14,23<br>18-22,26-30, 33,35,36 |
| X<br>Y | RAFIEE, P. et al., Activation of protein kinases in chronically hypoxic infant human and rabbit hearts: role in cardioprotection, Circulation, 2002, Vol.106, No.2, p.239-45, Abstract | 24,25<br>26-30 |
| X<br>Y | MÖLLHOFF, T. et al., Myocardial ischaemia in patients with impaired left ventricular function undergoing coronary artery bypass grafting--milrinone versus nifedipin, Eur.J. Anaesthesiol, 2002, Vol.19, No.11, pages 796 to 802, Abstract | 24,25<br>29,30 |
| X<br>Y | YOSHIOKA, Yasuhiro [Reprint Author] et al., Coactivation of protein kinase A and protein kinase C prevents H2O2-induced necrosis through ERK and p38 MAP kinase activation in PC12 cells., Journal of Pharmacological Sciences, 2004, Vol.94, No. Supplement 1, p.134P, full text | 31,32,34<br>33,35,36 |
| Y | CHEN, M. et al., Resistance to hydrogen peroxide induced injury in cardiomyocytes from PARP-1 deficient mice, FASEB Journal, April 2004, Vol.18, No.5, p.Abst. 709.5, full text | 21 |
| Y | GREEN, P.S. et al., Mitochondrial dysfunction is an early indicator of doxorubicin-induced apoptosis, Biochim.Biophys. Acta, 2002, Vol.1588, No.1, pages 94 to 101, Abstract | 21 |

Form PCT/ISA/210 (continuation of second sheet) (April 2005)

**INTERNATIONAL SEARCH REPORT**

International application No.
PCT/JP2005/017494

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br><br>A | EP 1449539 A2 (Mochida Pharmaceutical Co., Ltd.),<br>25 August, 2004 (25.08.04),<br>Example 2<br>& WO 98/18487 A1 & AU 9747267 A<br>& EP 992243 A1 & JP 10-520304 A<br>& US 2003/109416 A1 & EP 992243 B1<br>& DE 69730358 E | 40<br>1-4,6-11,41,<br>42,44<br>5,43 |
| Y<br><br>A | WO 87/06836 A (Emory University),<br>19 November, 1987 (19.11.87),<br>Example VII<br>& AU 8774840 A & EP 266419 A<br>& NO 8800141 A & PT 84880 A<br>& BR 8707308 A & FI 8800163 A<br>& US 4801452 A & DK 8800108 A<br>& JP 01-500592 A & HU 47431 T<br>& US 4837014 A & ES 2009264 A<br>& US 5017370 A & US 5028599 A<br>& EP 451880 A & US 5064643 A<br>& US 5078995 A & US 5080894 A<br>& US 5089260 A & CA 1297792 C<br>& IL 82519 A & EP 498470 A2<br>& EP 502554 A2 & NO 9202262 A<br>& EP 451880 A3 & US 5198211 A<br>& US 5240702 A & US 5240701 A<br>& US 5250294 A & EP 266419 A4<br>& EP 498470 A3 & EP 502554 A3<br>& JP 06-016571 A & JP 06-016562 A<br>& JP 06-016565 A & JP 06-016567 A<br>& EP 266419 B1 & JP 06-024993 A<br>& JP 94010139 B2 & JP 06-040924 A<br>& JP 06-048951 A & DE 3789196 G<br>& JP 06-100454 A & JP 94053685 B2<br>& JP 95035336 B2 & JP 95035337 B2<br>& JP 95035338 B2 & JP 95057730 B2<br>& FI 94928 B & EP 498470 B1<br>& DE 3751907 G | 1-4,6-11,41,<br>42,44<br>5,43 |
| Y<br><br>A | EP 1240906 A (Shionogi & Co., Ltd.),<br>18 September, 2002 (18.09.02),<br>Par. No. [0050]<br>& WO 2001/30387 A1 & AU 200076869 A<br>& KR 2002060207 A & JP 2001-532804 A<br>& CN 1411379 A & US 6924301 B1 | 1-4,6-11,41,<br>42,44<br>5,43 |
| Y<br><br>A | WO 2001/16137 A1 (Guilford Pharmaceuticals Inc.),<br>08 March, 2001 (08.03.01),<br>Page 69, lines 15 to 23<br>& AU 200070871 A & US 6291425 B1<br>& EP 1212328 A1 & JP 2003-508400 A<br>& MX 2002002213 A1 & US 6716828 B1<br>& US 2005/074470 A1 & AU 2005202592 A1 | 1-4,6-11,41,<br>42,44<br>5,43 |

Form PCT/ISA/210 (continuation of second sheet) (April 2005)

**INTERNATIONAL SEARCH REPORT**

International application No.
PCT/JP2005/017494

C (Continuation). DOCUMENTS CONSIDERED TO BE RELEVANT

| Category* | Citation of document, with indication, where appropriate, of the relevant passages | Relevant to claim No. |
|---|---|---|
| A | COLLIER, M.L. et al., Unitary chloride channels activated by protein kinase C in guinea pig ventricular myocytes, Circ.Res., 1995, Vol.76, No.2, pp.317-24, Abstract | 6-14,24,25, 31 |
| A | DUAN, Dayue [Reprint Author] et al., Loss of Early and Late Cardiac Ischemic Preconditioning in Cystic Fibrosis Transmembrane-Conductance Regulator Deficient Mice., FASEB Jouranal, 2004, Vol.18, No.4-5, p.Abst.429.6, full text | 6-14,24,25, 31 |
| A | CHEN, H. et al., Targeted inactivation of cystic fibrosis transmembrane conductance regulator chloride channel gene prevents ischemic preconditioning in isolated mouse heart. Circulation, Aug 2004, Vol.110, No.6, pp.700-4, Abstract | 6-14,24,25, 31 |
| A | KELLEY, T.J. et al., CFTR-mediated chloride permeability is regulated by type III phosphodiesterases in airway epithelial cells, Am.J.Respir Cell Mol.Biol., 1995, Vol.13, No.6, p.657-64, Abstract | 6-14,22,24, 25,31 |

Form PCT/ISA/210 (continuation of second sheet) (April 2005)

**INTERNATIONAL SEARCH REPORT**

International application No.
PCT/JP2005/017494

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 37-39
because they relate to subject matter not required to be searched by this Authority, namely:
Claims 37 to 39 pertain to [methods for treatment of the human body by therapy] and thus relate to a subject matter which this International Searching Authority is not required, under the provisions of the PCT Rule 39.1(iv), to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
"The special technical feature" of claims 1 to 11 resides in "an *in vivo* screening system provided with a thermal insulation unit, a ligation holder and a continuous drug injector"; "the special technical feature" of claims 12 to 39 relates to "a pharmaceutical composition for inhibiting cell death in ventricle muscle which contains a CFTR channel activator"; and "the special technical feature" of claims 40 to 44 resides in "an *in vivo* ischemia and postischemic reperfusion system provided with a thermal insulation unit". Since there is no technical relationship among these invention groups involving one or more of the same or corresponding special technical features, it does not appear that these invention groups are so (continued to extra sheet)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.
PCT/JP2005/017494

Continuation of Box No.III of continuation of first sheet(2)

linked as to form a single general inventive concept.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **J. L. PARK ; B. R. LUCCHESI.** Mechanisms of myocardial reperfusion injury. *Ann Thorac Surg,* 1999, vol. 68, 1905-1912
- **H. FURUKAWA ; M. HACHIDA ; K. YASUMOTO.** Myocardial preservation and reperfusion injury: Current trends and future perspectives. *Low Temp Med,* 2000, vol. 26, 93-98
- **N. HEDAYATI ; S. J. SCHOMISCH ; J. L. CARINO ; J. T. SHERWOOD ; E. J. LESNEFSKY ; B. L. CMOLIK.** Cardioprotection by St. Thomas' solution is mediated by protein kinase C and tyrosine kinase. *J Surg Bes,* 2003, vol. 113, 121-127
- **T. STEENSRUD ; D. NORDHAUG ; O. P. ELVENES ; C. KORVALD ; D. G. SORLIE.** Superior myocardial protection with nicorandil cardioplegia. *Eur J Cardjothorac Surg,* 2003, vol. 23, 670-677
- **T. STEENSRUD ; D. NORDHAUG ; K. V. HUSNES ; E. AGHAJANI ; D. G. SORLIE.** Replacing potassium with nicorandil in cold St. Thomas' Hospital cardioplegia improves preservation of energetics and function in pig hearts. *Ann Thorac Surg,* 2004, 1391-1397
- **W. J. LINZ ; A. E. BUSCH.** NHE-1 inhibition: from protection during acute ischaemia/reperfusion to prevention/reversal of myocardial remodelling. *Naunyn Schmiedehergs Arch Pharmacol,* 2003, vol. 368, 239-246
- **T. MINAMINO ; K. JIYOONG ; M. ASAKURA ; Y. SHINTANI ; H. ASANUMA ; M. KITAKAZE.** Rationale and design of a large-scale trial using nicorandil as an adjunct to percutaneous coronary intervention for ST-segment elevation acute myocardial infarction. Japan-working groups of acute myocardial infarction for the reduction of acute myocardial infarction for the reduction of necrotic damage by a K-ATP channel opener (J-WIND-KATP. *Circ J,* 2004, vol. 68, 101-106
- **N. N. AYE ; S. KOMORI ; K. HASHIMOTO.** Effects and interaction, of cariporide and preconditioning on cardiac arrhythmias and infarction in rat in vivo. *Br J Phalmacol,* 1999, vol. 127, 1048-1055
- **S. SANADA ; M. KITAKAZE ; P. J. PAPST ; H. ASANUMA ; K. NODE ; S. TAKASHIMA ; M. ASAKURA ; H. OGITA ; Y. LIAO ; Y. SAKATA.** Cardioprotective effect afforded by transient exposure to phosphodiesterase III inhibitors. *Circulation,* 2001, vol. 104, 705-710
- **M. P. RECHTMAN ; A. VAN DER ZYPP ; H. MAJEWSKI.** Amrinone reduces ischaemia-reperfusion injury in rat heart. *Eur J Pharmacol,* 2000, vol. 402, 255-262
- **A. F. RUMP ; D. ACAR ; W. KLAUS.** A quantitative comparison of functional and anti-ischaemic effects of the phosphodiesterase-inhibitors, amrinone, milrinone and levosimendan in rabbit isolated hearts. *Br J Pharmacol,* 1994, vol. 112, 757-762
- **H. URAMOTO ; S. MORISHIMA ; Y. ANDO-AKATSUKA ; Y. NAGASAKI ; N. HAGIWARA ; Y. OKADA.** A role of CFTR Cl- channel in the protection from ischemic injury in neonatal rat ventricular myocytes. *Jpn. J. Physiol.,* 2002, vol. 52, S35
- **NARUSE, S. ; KITAGAWA, M. ; ISHIGURO, H. ; FUJIKI, K. ; HAYAKAWA, T.** Cystic fibrosis and related diseases of the pancreas. *Best Pract. Res. Clin. Gastroenterol.,* 2002, vol. 16, 511-526
- **H. URAMOTO ; N. TAKAHASHI ; A. K. DUTTA ; R. Z. SABIROV ; Y. ANDO-AKATSUKA ; S. MORISHIMA ; Y. OKADA.** Ischemia-induced enhancement of CFTR expression on the plasma membrane in neonatal rat ventricular myocytes. *Jpn J Physiol,* 2003, vol. 53, 357-365
- **R. M. WALLIS ; J. D. CORBIN ; S. H. FRANCIS ; P. ELLIS.** Tissue distribution of phosphodiesterase families and the effects of sildenafil on tissue cyclic nucleotides, platelet function, and the contractile responses of trabeculae carneae and aortic rings in vitro. *Am J Cardiol,* 1999, vol. 83, 3C-12C
- **B. R. COBB ; L. FAN ; T. E. KOVACS ; E. J. SORSCHER ; J. P. CLANCY.** Adenosine receptors and phosphodiesterase inhibitors stimulate Cl- secretion in Calu-3 cells. *Am J Respir Cell Mol Biol,* 2003, vol. 29, 410-418
- **L. A. LEHTONEN ; S. ANTILA ; P. J. PENTIKAINEN.** Pharmacokinetics and pharmacodynamics of intravenous inotropic agents. *Clin Pharmacokinet,* 2004, vol. 43, 187-203
- **Y. OHIZUMI.** Novel types of cardiotonic drugs. *Folia Pharmacol Japon,* 1992, vol. 100, 259-269
- **Z. WANG ; T. MITUIYE ; S. A. REES ; A. NOMA.** Regulatory volume decrease of cardiac myocytes induced by β-adrenergic activation of the C1- channel in guinea pig. *J Gen Physiol,* 1997, vol. 10, 73-82